# EUROPEAN PATENT SPECIFICATION

(11) **EP 3 567 111 B1**
(45) Date of publication and mention of the grant of the patent: **09.07.2025**
(21) Application number: 18171637.4
(22) Date of filing: 09.05.2018
(51) Int. Cl.: C12N 15/82

(54) **GENE FOR RESISTANCE TO A PATHOGEN OF THE GENUS HETERODERA**
GEN FÜR RESISTENZ GEGEN EIN PATHOGEN DER GATTUNG HETERODERA
GÈNE DE RÉSISTANCE À UN PATHOGÈNE DU GENRE HETERODERA

(43) Date of publication of application: 13.11.2019
(73) Proprietor: KWS SAAT SE & Co. KGaA, 37574 Einbeck (DE)
(72) Inventor: TÖRJÉK, Ottó, 37574 Einbeck (DE); BORCHARDT, Dietrich, 37574 Einbeck (DE); MECHELKE, Wolfgang, 37574 Einbeck (DE); BEYER, Werner, 37574 Einbeck (DE); SCHULZ, Britta, 37574 Einbeck (DE); LEIN, Jens Christoph, 37085 Göttingen (DE)
(74) Representative: Michalski Hüttermann & Partner Patentanwälte mbB

(56) References cited:
- WO-A1-2014/127835
- M REUTHER,: "Nematode-tolerant sugar beet varieties - resistant or susceptible to the Beet Cyst Nematode Heterodera schachtii?", ZUCKERINDUSTRIE. SUGAR INDUSTRY, 1 April 2017 (2017-04-01), pages 277 - 284, XP055493551, Retrieved from the Internet <URL:https://www.researchgate.net/profile/Marie_Reuther/publication/316542022_Nematode-tolerant_sugar_beet_varieties_-_Resistant_or_J_Susceptible_to_the_Beet_Cyst_Nematode_Heterodera_schachtii/links/5902e5074585156502a31cec/Nematode-tolerant-sugar-beet-varieties-Resistant-or-J-Susceptible-to-the-Beet-Cys> [retrieved on 20180718], DOI: 10.1016/j.fcr.2011.02.007
- SANDRA HUNGER ET AL: "Isolation and linkage analysis of expressed disease-resistance gene analogues of sugar beet ( Beta vulgaris L.)", GENOME., vol. 46, no. 1, 1 February 2003 (2003-02-01), Ottawa; CA, pages 70 - 82, XP055493641, ISSN: 0831-2796, DOI: 10.1139/g02-106
- PIERGIORGIO STEVANATO ET AL: "Identification and Validation of a SNP Marker Linked to the Gene HsBvm-1 for Nematode Resistance in Sugar Beet", PLANT MOLECULAR BIOLOGY REPORTER., vol. 33, no. 3, 25 July 2014 (2014-07-25), NL, pages 474 - 479, XP055493557, ISSN: 0735-9640, DOI: 10.1007/s11105-014-0763-8
- KATHARINA SCHNEIDER ET AL: "Analysis of DNA polymorphisms in sugar beet (Beta vulgaris L.) and development of an SNP-based map of expressed genes", THEORETICAL AND APPLIED GENETICS ; INTERNATIONAL JOURNAL OF PLANT BREEDING RESEARCH, SPRINGER, BERLIN, DE, vol. 115, no. 5, 11 July 2007 (2007-07-11), pages 601 - 615, XP019535833, ISSN: 1432-2242, DOI: 10.1007/S00122-007-0591-4
- M BAKOOIE1: "Development of an SNP Marker for Sugar Beet Resistance/Susceptible Genotyping to Root-Knot Nematode", J. AGR. SCI. TECH., 1 January 2015 (2015-01-01), Berlin/Heidelberg, pages 443 - 454, XP055493555, Retrieved from the Internet <URL:http://ecopersia.modares.ac.ir/article-23-2636-en.pdf> [retrieved on 20180718], DOI: 10.1007/s00122-015-2611-0
- GINA G. CAPISTRANO-GOSSMANN ET AL: "Crop wild relative populations of Beta vulgaris allow direct mapping of agronomically important genes", NATURE COMMUNICATIONS, vol. 8, 6 June 2017 (2017-06-06), pages 15708, XP055387481, DOI: 10.1038/ncomms15708
- MAOQIAN WANG ET AL: "High-Density Genetic Map Construction in Sugar Beet (Beta vulgaris L.) by High-Throughput Technology", SUGAR TECH, vol. 20, no. 2, 9 September 2017 (2017-09-09), IN, pages 212 - 219, XP055492959, ISSN: 0972-1525, DOI: 10.1007/s12355-017-0550-6
- JULIANE C. DOHM ET AL: "The genome of the recently domesticated crop plant sugar beet (Beta vulgaris)", NATURE, vol. 505, no. 7484, 18 December 2013 (2013-12-18), GB, pages 546 - 549, XP055492884, ISSN: 0028-0836, DOI: 10.1038/nature12817
- BUTORINA A K ET AL: "Molecular genetic investigation of sugar beet (L.)", RUSSIAN JOURNAL OF GENETICS, NAUKA/INTERPERIODICA, MO, vol. 47, no. 10, 8 October 2011 (2011-10-08), pages 1141 - 1150, XP019962364, ISSN: 1608-3369, DOI: 10.1134/S102279541110005X
- SILKE MÖHRING ET AL: "Multiplexed, linkage group-specific SNP marker sets for rapid genetic mapping and fingerprinting of sugar beet (Beta vulgaris L.)", MOLECULAR BREEDING, KLUWER ACADEMIC PUBLISHERS, DO, vol. 14, no. 4, 1 January 2005 (2005-01-01), pages 475 - 488, XP019258693, ISSN: 1572-9788
- ADETUNJI IBRAHEEM ET AL: "Genetic diversity and linkage disequilibrium analysis in elite sugar beet breeding lines and wild beet accessions", THEORETICAL AND APPLIED GENETICS ; INTERNATIONAL JOURNAL OF PLANT BREEDING RESEARCH, SPRINGER, BERLIN, DE, vol. 127, no. 3, 1 December 2013 (2013-12-01), pages 559 - 571, XP035333111, ISSN: 0040-5752, [retrieved on 20131201], DOI: 10.1007/S00122-013-2239-X
- CAI D ET AL: "POSITIONAL CLONING OF A GENE FOR NEMATODE RESISTANCE IN SUGAR BEET", SCIENCE, AMERICAN ASSOCIATION FOR THE ADVANCEMENT OF SCIENCE, US, vol. 275, 7 February 1997 (1997-02-07), pages 832 - 834, XP002055588, ISSN: 0036-8075, DOI: 10.1126/SCIENCE.275.5301.832
- TIM THURAU ET AL: "Plant Nematode Control", SUGAR TECH, vol. 12, no. 3-4, 1 December 2010 (2010-12-01), pages 229 - 237, XP055060036, ISSN: 0972-1525, DOI: 10.1007/s12355-010-0056-y

## Description

### FIELD OF THE INVENTION

The present application relates to a nucleic acid molecule which when present in a plant is able to confer a resistance to a pathogen of the genus Heterodera - in particular, to the beet cyst nematode *Heterodera schachtii,* and, in particular, in a plant of the species *Beta vulgaris* - as well as to the polypeptide encoded by the nucleic acid molecule according to the invention. In particular, the nucleic acid molecule according to the present application is characterized in that the resistance effect toward pathogens of the genus Heterodera that is conferred by the presence of the nucleic acid molecule is dominant. Furthermore, the present application relates to a Heterodera-resistant plant, plant cell, plant organ, plant tissue, plant part, or a seed or descendant of a plant, which comprises the nucleic acid molecule or portions thereof as an endogenous gene, as an edited gene, or as a transgene. Furthermore, the present application also discloses methods for increasing the resistance toward a pathogen of the genus Heterodera, in a plant, in particular in a plant of the species *Beta vulgaris,* as well as methods for producing or identifying and possibly selecting a Heterodera-resistant plant. The present application also discloses methods for monitoring an infestation by the pathogen *Heterodera schachtii,* as well as oligonucleotide probes and primers for hybridization with the nucleic acid molecule according to the invention.

### BACKGROUND OF THE INVENTION

In commercially grown sugar beets, more than two dozen different nematode species have been reported to cause economic damage (Hafez, Sugar Beet nematodes in Idaho and Eastern Oregon (1997), University of Idaho, College of Agriculture). The most serious nematode pest of beet is the beet cyst nematode (*Heterodera schachtii*) (Cooke, Agricultural Zoology Reviews 2 (1987), 132-183) which has the highest economic importance in most German and European beet growing areas. It was first detected in 1859 in Germany, and based on an estimation currently 10-25% of the sugar beet production areas could be infested with this pest worldwide causing yield losses up to 80% (Hafez 1997), wherein the yield loss depends on the amount of nematodes in the soil, the sowing and infestation time of the sugar beets as well as on the weather conditions.

The beet cyst nematode is a plant pathogenic nematode and can cause considerable yield loss not only in sugar beets but also in other beets such as red beet, fodder beet and chard, as well as in other plants of the family Amaranthaceae, such as spinach, and Brassicacea, such as rape seed, cabbages, Chinese cabbages, cauliflowers, Brussels sprouts, broccoli, turnip, radish and swede, by severely damaging root systems, especially during summer. This nematode also infects many common weeds such as wild turnip, shepherd's purse, fat-hen and portulaca.

In sugar beet fields, beet cyst nematode infestation initially appears as circular to oval areas of stunted plants. Nematodes feed on plant roots, reducing the plant's ability to take up nutrients and water. Therefore above-ground symptoms look like nutrient deficiency or drought, reduced stand, poor growth, stunting, yellowing and wilting, wherein the symptoms vary based on growth stage at the time of infection. When seedlings are infected, symptoms include stunting and reduced leaf growth, and older outer leaves become yellow and wilted during the hot period of the day. An infested crop contains smaller plants of reduced value and quality and will compete poorly with weeds.

The spread of the disease is continuous and the pest has become increasingly difficult for growers to manage. However, it is crucial to control the pest, as high soil nematode populations can make production of sugar beets uneconomical. Different methods to fight back against the disease exist, but none of the currently applied practices is satisfactory. A chemical control of *Heterodera schachtii* via nematicides not only incurs costs to the farmer and pollutes the environment but is also no longer allowed in many countries, whereas soil decontamination is not applicable on larger fields. Furthermore, crop rotation wherein sugar beets are only grown every four years or even less frequent in order to reduce the population of the nematodes is not always practicable and not effective enough. A further common management practice includes the cultivation of nematode resistant catch-crops such as oil radish or mustard. These plants attract the pest but inhibit its development and reproduction, which reduces the pest population. It is also possible to grow resistant or tolerant sugar beet varieties. So far the most effective method for decreasing the nematode soil population was growing resistant sugar beet varieties.

Meanwhile nematode resistant and tolerant sugar beet cultivars are offered on the marked harboring for example a major resistance gene against Heterodera schachtii from Beta procumbens (Heijbroek et al., Euphytica 38 (1988), 121-131; Lange et al., Proceedings of the 53rd IIRB Congress, Brussels (1990), 89-102). In the translocated *Beta procumbens* segment, *i.e. B. procumbens* chromosome 1 segment integrated at the end of *B. vulgaris* chromosome 9, an Hs1pro-1 gene was identified by positional cloning as a causal factor (Cai et al., Science 275 (1997), 832-834). However, when integrating the *Beta procumbens* chromosome 1 segment into the genome of *Beta vulgaris* not only is the desired resistance to *Heterodera schachtii* introduced into the plant, but, rather, often unwanted features as well, such as, for example, reduced yield, due to the inheritance of additional genes that are linked with the positive feature of Heterodera resistance. This phenomenon is also known by the term, "linkage drag." Thus, the use of this gene in breeding has limitations due to a significant yield penalty because of linkage drag and in addition due to the instability of the translocation.

A further source of nematode resistance was found in the wild sea beet *B. vulgaris* subsp. *maritima* in material collected in France (Hijner, Meded. Inst. rat. Suikerprod. 21 (1951), 1-13). However, the genetic and functional background of Heterodera resistance and the identity of the resistance genes have until now been entirely unclear.

However, as mentioned above, a disadvantage of cultivars having the described resistances consists in the cultivar development being very laborious and complicated due to the complicated heredity, and in such cultivars having a markedly poorer yield performance relative to normal cultivars, in the absence of an infestation. Among other things, this may be linked to the epigenetic interaction of some resistance genes with genes that are responsible for sugar production, which leads to reduced fitness of the plants, in the absence of the pathogen.

The use of new breeding techniques based upon gene editing, *e.g.,* by means of TALE nucleases or CRISPR systems, and of transgenic approaches, is impossible in practice since the genes which are involved in the resistance development have not been identified and characterized.

For sustainable breeding against Beet cyst nematode that is to counteract the danger of Heterodera variants that overcome resistance, it is necessary to continuously identify new resistance genes and integrate these into the gene pools of cultivated plants such as sugar beets. In particular, the aim consisted in the provision of suitable resistance genes that, when present in the plant, on their own already produce a very large, dominant resistance effect against *Heterodera schachtii.* According to the invention, this aim is achieved via the embodiments characterized in the claims.

### DESCRIPTION OF THE INVENTION

The invention is defined by the features of the independent claims.

The present application relates to a nucleic acid molecule that is able to confer a resistance towards a pathogen of the genus Heterodera - in particular, to the beet cyst nematode *Heterodera schachtii* - in a plant, and, in particular, in *Beta vulgaris* subsp. *vulgaris.* The nucleic acid molecule, when present in the plant produces a dominant resistance effect against *Heterodera schachtii.*

Furthermore, the present application relates to a Heterodera-resistant plant, plant cell, plant organ, plant tissue, plant part, a seed, seed stock, or descendant of a plant, which endogenously or transgenically comprises the nucleic acid molecule or portions thereof. According to present application, those plants and their components that have been obtained exclusively by means of an essentially biological process are exempted.

Methods for increasing the resistance to Heterodera in a plant, in particular in a plant of the species *Beta vulgaris,* as well as methods for producing or identifying and possibly selecting a Heterodera-resistant plant, are likewise disclosed in the present application. The present application also relates to methods for monitoring an infestation of the pathogen *Heterodera schachtii,* as well as oligonucleotides as probes and primers for hybridization with the nucleic acid molecule according to the invention.

The present invention relates to
[1] a nucleic acid molecule for increasing the resistance towards a nematode of the genus Heterodera in a Beta vulgaris plant in which the nucleic acid molecule is expressed thereby characterized that the nucleic acid molecule is selected from the group consisting of:
   (a) a nucleotide sequence which comprises the sequence selected from the group consisting of SEQ ID NOs. 1, 4 and 7;
   (b) a nucleotide sequence which comprises the coding sequence selected from the group consisting of SEQ ID NOs. 2, 5 and 8;
   (c) a nucleotide sequence which hybridizes with a complementary sequence of a nucleotide sequence according to (a), (b), (f) or (g) under stringent conditions;
   (d) a nucleotide sequence which comprises a sequence that is at least 90% identical to the sequence of the nucleotide sequence of any one of (a) or (b);
   (e) a nucleotide sequence encoding a polypeptide having an amino acid sequence selected from the group consisting of SEQ ID NOs. 3, 6 and 9;
   (f) a nucleotide sequence encoding a polypeptide having an amino acid sequence that is at least 94% identical to the amino acid sequence selected from the group consisting of SEQ ID NOs. 3, 6 and 9;
   (g) a nucleotide sequence which is a variant of a DNA sequence of any of (a) to (f) due to the degeneracy of the genetic code, wherein stringent conditions are defined as hybridization in 4 x SSC at 65°C, and subsequent repeated washing in 0.1 x SSC at 65°C for approximately 1 hour in total.
   The present application discloses
[2] a nucleic acid molecule according to [1], characterized in that the nucleic acid molecule confers a resistance towards a pathogen of the genus Heterodera that is dominant in a plant,
[3] a nucleic acid molecule according to [1] or [2], characterized in that the nucleic acid molecule originates from *Beta vulgaris* subsp. *maritima.*
[4] The present invention relates to a polypeptide which is encoded by the nucleic acid molecule according to [1].
[5] The present invention relates to a vector or expression cassette comprising the nucleic acid molecule according to [1]. The nucleic acid molecule is preferably heterologous to the vector or expression cassette or the nucleic acid molecule is preferably linked to a heterologous regulatory element, preferably a promoter or terminator.
[6] The present invention relates to a cell which comprises the nucleic acid molecule according to [1] as a transgene, or the vector or expression cassette according to [5].
[7] The present application discloses
   a plant or a portion thereof, characterized in that the plant or its portion contains the nucleic acid molecule according to one of [1] through [3] endogenously or transgenically, or the vector or expression cassette according to [5], wherein preferably the plant which endogenously contains the nucleic acid molecule is a plant of the genus Beta, in particular of the species *Beta vulgaris,* - but *not Beta vulgaris* subsp. *maritima.* Preferably said plant is a plant having a resistance towards a pathogen of the genus Heterodera,
[8] a plant according to [7], characterized in that the plant is a hybrid plant,
[9] a plant according to [7] or [8], characterized in that the nucleic acid molecule is present heterozygously or homozygously in the genome of the plant.
[10] The present application discloses seeds or descendants of the plant according to one of [7] through [9], wherein the seed or the descendant transgenically or endogenously comprises the nucleic acid molecule according to one of [1] through [3], or the vector or expression cassette according to [5].
[11] The present invention relates to a seed of a Beta vulgaris plant, characterized in that the plant is not *B. vulgaris* subsp. *maritima,* wherein the seed comprises the nucleic acid molecule according to [1], wherein the seed has been derived from a plant produced by the method according to [13] to [15] or wherein the seed is not a seed of a plant exclusively obtained by means of an essentially biological process, optionally wherein the seed has been technically treated, whereby the technical treatment is selected from the group consisting of:
   (a) Polishing
   (b) Dressing preferably pelleting
   (c) Incrustation
   (d) Colouring.
[12] The present invention relates to a method for increasing the resistance to a nematode of the genus Heterodera in a Beta vulgaris plant, including the following steps:
   (i) integration of the nucleic acid molecule according to [1] by means of homology-directed repair - preferably, supported by site-directed nuclease - into the genome of at least one cell of a plant, and optional regeneration of a plant from the plant cell; or
   (ii) increase in the expression of the nucleic acid molecule according to [1] in the plant by modification of the native promoter or by fusion of the nucleic acid molecule with a heterologous promoter that exhibits a higher activity in comparison to the native promoter; or
   (iii) transformation of a plant cell with the nucleic acid molecule according to [1], or the vector or the expression cassette according to [5], and regeneration of the transgenic plant from the transformed plant cell.
[13] The present invention relates to a method for producing a Beta vulgaris plant having resistance towards a nematode of the genus Heterodera, including the following steps:
   (a) transformation of a plant cell with the nucleic acid molecule according to [1], or the vector or the expression cassette according to [5]; and
   (b) regeneration of the transgenic plant from the transformed plant cell; or
      (i) introduction of a site-directed nuclease and a repair matrix into a cell of a plant, wherein the site-directed nuclease is able to generate at least one single-strand break or at least one double-strand break of the DNA in the genome of the cell preferably, upstream and/or downstream of a target region and the repair matrix comprises the nucleic acid molecule according to [1];
      (ii) cultivation of the cell from (i) under conditions that allow a homology-directed repair or a homologous recombination, wherein the nucleic acid molecule is integrated from the repair matrix into the genome of the plant; and
      (iii) regeneration of a plant from the cell modified in (ii)..
[14] The present invention relates to a method according to [13], characterized in that the target region
   a) is located between a marker according to SEQ ID NO: 10 and a marker according to SEQ ID NO: 11, or
   b) is flanked by a marker according to SEQ ID NO: 10 and a marker according to SEQ ID NO: 11, or
   c) comprises a chromosomal interval between a marker according to SEQ ID NO: 10 and a marker according to SEQ ID NO: 11.
[15] The present invention relates to a method according to [13] or [14], characterized in that the at least one single-strand break or the at least one double-strand break occurs at a position that is at most 10,000 base pairs upstream and/or downstream of the target region.
[16] The present application discloses a plant or portion thereof, obtained or obtainable by a method according to one of [13] to [15].
[17] The present invention relates to a method for identifying, and optionally providing or selecting, a Beta vulgaris plant that is resistant toward a nematode of the genus Heterodera, characterized in that the method includes at least step (i) or (ii):
   (i) detection of the presence and/or expression of the nucleic acid molecule according to [1], or the presence of the polypeptide according to [4], in the Beta vulgaris plant or a portion of the Beta vulgaris plant; and/or
   (ii) detection of at least one region which is located between the marker according to SEQ ID NO 10 and the marker according to SEQ ID NO 11, is flanked by the marker according to SEQ ID NO 10 and the marker according to SEQ ID NO 11, or comprises a chromosomal interval between the marker according to SEQ ID NO 10 and the marker according to SEQ ID NO 11, co-segregating with the nucleotide sequence of the nucleic acid molecule(s) according to [1]; and
   (iii) optional selection of the Beta vulgaris plant having resistance towards a nematode of the genus Heterodera
   wherein the marker according to SEQ ID NO 10 is a single nucleotide polymorphism (SNP) at position 56940072 bp of chromosome 5 referenced to Beta vulgaris genotype EL10, wherein said nucleotide is G or T, and the marker according to SEQ ID NO 11 is a single nucleotide polymorphism (SNP) at position 57809807 bp of chromosome 5 referenced to Beta vulgaris genotype EL10, wherein said nucleotide is G or T.
[18] The present application discloses a method for identification of a nucleic acid molecule which when present in the plant is able to confer a resistance towards a pathogen of the genus Heterodera in a plant, preferably in a plant of the species *Beta vulgaris,* characterized in that the method includes the following steps:
   (i) comparison of the amino acid sequence of the polypeptide according to [4] with amino acid sequences from a sequence database, or identification of allelic variants which encode the polypeptide according to [4] in genotypes of the plant;
   (ii) identification of the amino acid sequence, or an allelic variant, encoding an amino acid sequence, wherein the amino acid sequence is at least 70%, at least 75%, at least 80%, at least 85%, at least 90%, at least 92%, at least 94%, at least 96%, at least 97%, at least 98%, or at least 99% identical to the amino acid sequence of the polypeptide according to [4];
   (iii) introduction of a nucleic acid molecule, or the allelic variant, encoding the identified amino acid sequence into a plant, preferably into a plant of the species *Beta vulgaris,* and expression of the nucleic acid molecule in the plant; and
   (iv) detection of the resistance towards a pathogen of the genus Heterodera.
[19] The present application discloses a method for cultivation of plants, preferably of plants of the species *Beta vulgaris,* including
   (i) the provision of plants according to one of [7] through [9], the production of plants with the aid of a method according to one of [13] through [16], or the identification and selection of plants with the aid of a method according to [17], and
   (ii) cultivation of the plants from (i) or descendants thereof,
   wherein the method counteracts an infestation of the cultivated plants with a pathogen of the genus Heterodera.
[20] The present application discloses oligonucleotide of at least 15, 16, 17, 18, 19, or 20 - preferably, at least 21, 22, 23, 24, or 25, particularly preferably, at least 30, 35, 40, 45, or 50, and, particularly preferably, at least 100, 200, 300, or 500 - nucleotides in length, which oligonucleotide specifically hybridizes with a nucleotide sequence as defined in one of [1] through [3].
[21] The present application discloses a pair of oligonucleotides - preferably, oligonucleotides according to [20] or a kit containing these oligonucleotides - wherein the oligonucleotides are suitable for hybridization as forward primer and reverse primer to a region in the *Beta vulgaris* genome that, co-segregates in *Beta vulgaris* with the resistance resistance towards a pathogen of the genus Heterodera conferred by the nucleic acid molecule according to one of [1] through [3], preferably wherein the region in the *Beta vulgaris* genome is located between marker s5e3001s02 and marker s5e4668xxx, is flanked by marker s5e3001s02 and marker s5e4668xxx, or comprises a chromosomal interval between marker s5e3001s02 and marker s5e4668xxx.
[22] The present invention relates to the use of an oligonucleotide primer of at least 20 nucleotides in length, which oligonucleotide specifically hybridizes as probe with any of the nucleotide sequences selected from the group consisting of
   (i) a nucleotide sequence which comprises the sequence selected from the group consisting of: SEQ ID NO 1, 4 and 7;
   (ii) a nucleotide sequence which comprises the coding sequence selected from the group consisting of: SEQ ID NO 2, 5 and 8;
   (iii) a nucleotide sequence encoding a polypeptide having an amino acid sequence selected from the group consisting of: SEQ ID NO 3, 6 and 9,
   for amplifying of a region specific to the nucleic acid molecule of [1] in a polymerase chain reaction.
[23] The present application discloses a method, plant, or plant portion or pair of oligonucleotides according to any of the preceding items, wherein
   s5e3001s02 is a single nucleotide polymorphism (SNP), preferably at position 56940072 bp of chromosome 5 referenced to Beta vulgaris genotype EL10, wherein said nucleotide is G or T, preferably a single nucleotide polymorphism (SNP) as set forth in SEQ ID NO: 10, more preferably said nucleotide is T; and/or
   s5e4668xxx is a single nucleotide polymorphism (SNP), preferably at position 57809807 bp of chromosome 5 referenced to Beta vulgaris genotype EL10, wherein said nucleotide is G or T, preferably a single nucleotide polymorphism (SNP) as set forth in SEQ ID NO: 11, more preferably said nucleotide is T.
[24] The present application discloses the plant according to [7] wherein the plant or a pelleted seed of such a plant has a genome allowing the development of a beet body having a minimum fresh mass of 200g, 250g, 300g, 350g, 400g, 450g or 500g and a maximum mass of 100g, 1100g, 1200g, 1300g, 1400g, 1500g, 1600g, 1700g, 1800g, 1900g or 2000g.
[25] The present application discloses the plant according to [7] or [24] a sugar beet plant or a pelleted seed of such a plant wherein the genome of the sugar beet plant allows development of a beet body having a saccharose concentration in the fresh mass of the beet body of at least 10%, 11%, 12%, 13%, 14%, 15%, 16%, 17%, 18%,19% or even 20% (percent by mass).

First, some of the terms used in this application are explained in detail in the following:
The genus *Heterodera* encompasses various species, *e.g.,* the species *Heterodera amygdali, Heterodera arenaria, Heterodera aucklandica, Heterodera avenae, Heterodera bergeniae, Heterodera bifenestra, Heterodera cacti, Heterodera cajani, Heterodera canadensis, Heterodera cardiolata, Heterodera carotae, Heterodera cicero, Heterodera cruciferae, Heterodera delvii, Heterodera elachista, Heterodera filipjevi, Heterodera gambiensis, Heterodera glycines, Heterodera goettingiana, Heterodera hordecalis, Heterodera humuli, Heterodera latipons, Heterodera longicaudata, Heterodera medicaginis, Heterodera oryzae, Heterodera oryzicola, Heterodera rosii, Heterodera rostochiensis, Heterodera sacchari, Heterodera schachtii, Heterodera tabacum, Heterodera trifolii, Heterodera ustinovi* and *Heterodera zeae.*

In conjunction with the specification of a length of a nucleotide sequence, the term, "approximately," means a deviation by +/- 200 base pairs - preferably, by +/- 100 base pairs, and, particularly preferably, by +/- 50 base pairs.

A "plant of the genus Beta" belongs to the amaranth family (Amaranthaceae). Numbering among these plants are plants of the species *Beta macrocarpa, Beta vulgaris, Beta lomatogona, Beta macrorhiza, Beta corolliflora, Beta trigyna,* and *Beta nana.* A plant of the species *Beta vulgaris* is, in particular, a plant of the subspecies *Beta vulgaris* subsp. *vulgaris.* For example, numbering among these are *Beta vulgaris* subsp. *vulgaris* var. *altissima* (sugar beet in a narrower sense), *Beta vulgaris* ssp. *vulgaris* var. *vulgaris* (chard), *Beta vulgaris* ssp. *vulgaris* var. *conditiva* (beetroot / red beet), *Beta vulgaris* ssp. *vulgaris* var. *crassa*/*alba* (fodder beet). It is noted that the nucleic acid according to the invention does not naturally occur in sugar beet, chard, beetroot, or fodder beet, but may be introduced into these via human action.

A "functional fragment" of a nucleotide sequence means a segment of a nucleotide sequence which has a functionality identical or comparable to that of the complete nucleotide sequence from which the functional fragment originates. As such, the functional fragment may possess a nucleotide sequence which is identical or homologous to the total nucleotide sequence over a length of at least 50%, 55%, 60%, 65%, 70%, 75%, 80%, 85%, 90%, 92%, 94% 96%, 97%, 98%, or 99%. This also explicitly encompasses the range of 90 - 100%. Furthermore, a "functional fragment" of a nucleotide sequence may also mean a segment of a nucleotide sequence which modifies the functionality of the entire nucleotide sequence, e.g., in the course of post-transcriptional or transcriptional gene silencing. As such, the functional fragment of a nucleotide sequence may comprise at least 14, 15, 16, 17, 18, 19, 20, 21, 22, 23, 24, or 25 - preferably, at least 30, 35, 40, 45, 50, 60, 70, 80, 90, 100, 120, or 140, and, particularly preferably, at least 160, 180, 200, 250, 300, 350, 400, 450, 500, 600, 700, 800, 900, or 1,000 - successive nucleotides of the total nucleotide sequence. This also explicitly encompasses the range of 21 to 50 nucleotides.

A "functional part" of a protein means a segment of a protein, or a section of the amino acid sequence, that encodes the protein, wherein the segment may exert functionality identical or comparable to that of the entire protein in a plant cell. Over a length of at least 50%, 55%, 60%, 65%, 70%, 75%, 80%, 85%, 90%, 92%, 94% 96%, 97%, 98%, or 99%, a functional part of a protein has an amino acid sequence that is identical or, with consideration of conservative and semi-conservative amino acid exchanges, similar to the protein from which the functional part originates.

The term, "heterologous," means that the introduced polynucleotide originates from a cell or an organism with a different genetic background, of the same species or a different species, or is homologous to the prokaryotic or eukaryotic host cell, but is then located in a different genetic environment and thus differs from a corresponding polynucleotide that is possibly naturally present. A heterologous polynucleotide may be present in addition to a corresponding endogenous gene.

In the sense of the invention, what is understood by a "homolog" is a protein of the same phylogenetic origin; what is understood by an "analog" is a protein which exerts the same function, but has a different phylogenetic origin; what is understood by an "ortholog" is a protein from a different species that exerts the same function; and what is understood by a "paralog" is a protein that has appeared within a species due to duplication, wherein this copy either retains the same protein function, alters its expression template, but not the function, changes its protein function, or divides up the original gene function between both copies.

What is to be understood by "hybridizing" or "hybridization" is a process in which a single-stranded nucleic acid molecule binds to a nucleic acid strand that is complementary to the greatest possible extent, i.e., forms base pairs with this. Standard methods for hybridization are described in, for example, Sambrook et al., Molecular Cloning: A Laboratory Manual, 3rd ed., Cold Spring Harbor Laboratory Press, Cold Spring Harbor, NY, 2001. What is preferably understood by this is that at least 60% - more preferably, at least 65%, 70%, 75%, 80%, or 85%, and, particularly preferably, 90%, 91%, 92%, 93%, 94%, 95%, 96%, 97%, 98%, or 99% - of the bases of the nucleic acid molecule form a base pairing with the nucleic acid strand that is complementary to the greatest possible extent. The possibility of such an annealing depends upon the stringency of the hybridization conditions. The term, "stringency," relates to the hybridization conditions. High stringency is present when a base pairing is made more difficult; low stringency is present if a base pairing is made easier. For example, the stringency of the hybridization conditions depends upon the salt concentration or ionic strength and the temperature. In general, the stringency may be increased by increasing the temperature and/or decreasing the salt content. What are to be understood by "stringent hybridization conditions" are those conditions given which a hybridization predominantly occurs only between homologous nucleic acid molecules. The term, "hybridization conditions," thereby relates not only to the conditions prevailing in the actual addition of the nucleic acids, but also to the conditions prevailing in the following washing steps. For example, stringent hybridization conditions are conditions under which, predominantly, only those nucleic acid molecules hybridize that have at least 70% - preferably, at least 75%, at least 80%, at least 85%, at least 90%, or at least 95% - sequence identity. Stringent hybridization conditions are, for example: hybridization in 4 x SSC at 65 °C, and subsequent repeated washing in 0.1 x SSC at 65 °C for approximately 1 hour in total. A hybridization preferably occurs under stringent conditions.

In relation to a nucleic acid in the form of a double-stranded DNA, "complementary" nucleotide sequence means that the second DNA strand complementary to the first DNA strand has the nucleotides that correspond to the bases of the first strand, in accordance with the base pairing rules. A complementary sequence is, preferably, entirely complementary to the counter-sequence, and thus preferably has the same length.

What is understood by an "isolated nucleic acid molecule" is a nucleic acid molecule extracted from its natural or original environment. The term also encompasses a synthetically-produced nucleic acid molecule. What is understood by an "isolated polypeptide" is a polypeptide extracted from its natural or original environment. The term also encompasses a synthetically produced polypeptide.

A "molecular marker" is a nucleic acid that is polymorphic in a plant population and is used as a reference or orientation point. A marker for the detection of a recombination event should be suitable for monitoring differences or polymorphisms within a plant population. Such a marker is thus able to detect and differentiate between various allelic states (alleles). The term, "molecular marker," also relates to nucleotide sequences which are complementary or at least largely complementary or homologous to genomic sequences - for example, nucleic acids which are used as probes or primers. These differences at the DNA level are to be found as markers and are, for example, polynucleotide sequence differences, *e.g.,* SSR's (*simple sequence repeats*), RFLP's (*restriction fragment length polymorphisms*), FLP's (*fragment length polymorphisms*) or SNP's (*single nucleotide polymorphisms*). The markers may be derived from genomic or expressed nucleic acids, e.g., spliced RNA, cDNA, or EST's, and may also relate to nucleic acids that are used as probes or primer pairs and as such are suitable for amplifying a sequence fragment using PCR-based methods. Markers that describe genetic polymorphisms (between parts of a population) may be detected using well-established methods from the prior art (An Introduction to Genetic Analysis, 7th edition, Griffiths, Miller, Suzuki, et al., 2000). For example, among these are DNA sequencing, PCR-based, sequence-specific amplification, verification of RFLP's, verification of polynucleotide polymorphisms by means of allele-specific hybridization (ASH), detection of amplified variable sequences of the plant genome, detection of a 3SR (*self-sustained sequence replication*), detection of SSR's, SNP's, RFLP's, or AFLP's (*amplified fragment length polymorphisms*). Furthermore, the methods for detection of EST's (*expressed sequence tags*) and SSR markers derived from EST sequences and RAPD (*randomly amplified polymorphic DNA*) are also known. Depending upon the context, the term, "marker," in the description may also mean a specific chromosome position in the genome of a species where a specific marker (SNP, for example) may be found.

Markers also include synthetic oligonucleotides that may be connected with one or more detection molecules, wherein the detection molecules may be used for a detection reaction or the generation of a signal within the scope of a verification method. Synthetic oligonucleotides also include labeled primers. Labeled primers are artificial compounds, do not occur in nature, and cannot be isolated from nature. The production of such compounds is explained further below.

A "promoter" is a non-translated, regulatory DNA sequence, typically upstream of a coding region, which contains the binding point for the RNA polymerase and initiates the transcription of the DNA. A promoter additionally contains other elements that act as a regulator gene for gene expression (for example, cis-regulatory elements). A "core or minimal promoter" is a promoter that has the basic elements which are needed for transcription initiation (for example, TATA box and/or initiator).

A "pathogen" means an organism that, in interactions with a plant, leads to disease symptoms in one or more organs in the plant. As used herein, pathogen means a nematode, in particular a nematode of the genus Heterodera.

What is to be understood by a "pathogenic infection" is the earliest point in time at which a pathogen interacts with a plant host tissue. In this sense, "infestation" means the occurrence of contact between pathogen and host. In the case of *Heterodera schachtii,* cysts are activated in the soil, juveniles will hatch when the development to second stage juveniles is completed and infest the roots of host plants. The nematodes penetrate the elongation zone behind the root tip and initiate the transformation of root cells to syncytia (specialized feeding structures). Syncytia concurrently increase with the nematode development to adults and may lead to impaired root functioning, which limits crop performance and results in yield losses. Without host plants, *Heterodera schachtii* can outlast within cysts in the soil for years.

Plant "organs" means, for example, leaves, shoot, stem, roots, hypocotyl, vegetative buds, meristems, embryos, anthers, ovula, seeds, or fruits. "Plant parts" include, but are not limited to, the shoot or the stalk, leaves, blossoms, inflorescence, roots, fruits, and seeds, as well as the pollen. The term, "plant parts," also means an association of multiple organs, *e.g.,* a blossom or a seed, or a part of an organ, *e.g.,* a cross-section through the plant shoot. Plant "tissues" are, for example, callus tissue, storage tissue, meristematic tissue, leaf tissue, shoot tissue, root tissue, plant tumor tissue, or reproductive tissue, as well as the cambium, parenchyma, vascular tissue, sclerenchyma, and epidermis. However, the tissue is not limited to this listing. For example, what are to be understood by plant "cells" are, for example, isolated cells having a cell wall or aggregates thereof, or protoplasts.

**In** conjunction with the present invention, the term, "regulatory sequence," relates to a nucleotide sequence which influences the specificity and/or the expression strength, *e*.*g*., in that the regulatory sequence confers a defined tissue specificity. Such a regulatory sequence may be located upstream of the transcription initiation point of a minimal promoter, but also downstream thereof, *e.g.,* in a transcribed, but not translated, leader sequence or within an intron. The term "regulatory sequence" can also encompass a whole promoter or a cis-element that is suitable to be used within a promoter.

The term, "resistance" is to be understood broadly and covers the range of the protection from a retardation up to a complete blocking of the development of the disease. One example of an important pathogen is *Heterodera schachtii.* A resistant plant cell of the invention or resistant plant of the invention preferably achieves a resistance to *Heterodera schachtii* which is defined as the ability of a plant to limit nematode multiplication. For example, an increase in the resistance can be measured via taking soil samples and determining the amount of nematodes and/or via determining the amount of cysts formed on the roots of the plants.

"Transgenic plant" relates to a plant into whose genome is integrated at least one polynucleotide. It may thereby be a heterologous polynucleotide or an exogenous polynucleotide. The polynucleotide is, preferably, stably integrated, which means that the integrated polynucleotide is stably preserved in the plant, is expressed, and also may be stably passed on to the descendants. The stable introduction of a polynucleotide into the genome of a plant also includes the integration into the genome of a plant of the preceding parental generation, wherein the polynucleotide may be stably passed on further. The term, "heterologous," means that the introduced polynucleotide originates from a cell or an organism with a different genetic background wherein the cell or the organism may belong to the same species or a different species, or is homologous to the prokaryotic or eukaryotic host cell, for example, but then is located in a different genetic environment and thus differs from a corresponding polynucleotide that is possibly naturally present. A heterologous polynucleotide may be present in addition to a corresponding endogenous gene.

As used herein, plant means any dicotyledonous or monocotyledonous plant, in particular a plant of the family Amaranthaceae, such as *Beta vulgaris* and *Spinacia oleracea,* and Brassicacea, such as *Brassica napus, Brassica oleracea, Brassica rapa, Raphanus sativus, Brassica juncacea, Brassica nigra, Eruca vesicaria subsp. sativa.*

Designs and embodiments are described by way of example with reference to the pending sequences and figures.

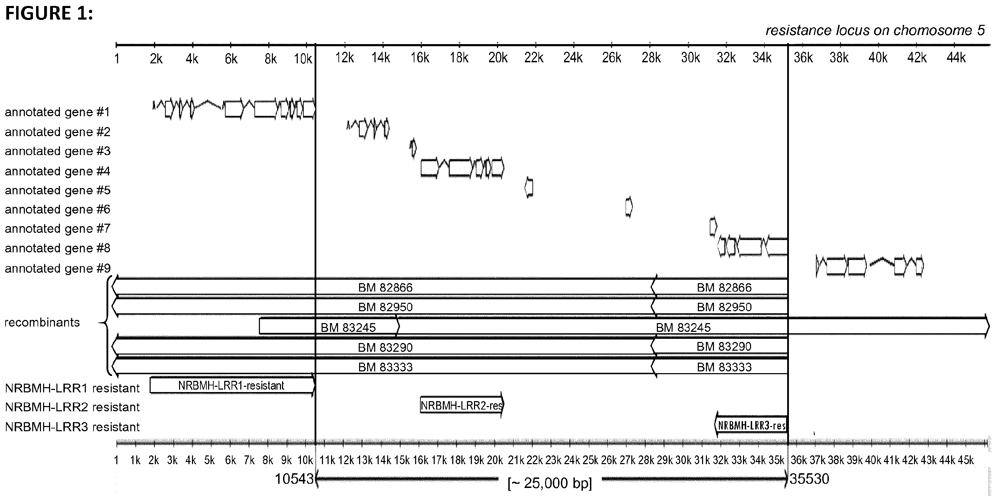

Fig. 1 Illustration of the sequence mapping and assembling within the target region on chromosome 5 (x-axis: physical distance in thousands of base pairs (1k = 1000bp)):
Upper part = Assembling of nine annotated genes (#1 -#9); the direction of the arrowheads symbolizes the 5'-3'-direction of each putative gene.
Middle part = illustrations of different genetic introgressions of accessions from *B. vulgaris* subsp. *maritima* (BM) recombination events are indicated according to their localization.
Bottom part = physical mapping of the candidate genes LRR1, LRR2 and LRR3 according to SEQ ID Nos 1, 4 and 7.

### DETAILED DESCRIPTION OF THE INVENTION

The present application relates to a nucleic acid molecule that is able to confer a resistance towards a pathogen of the genus Heterodera when present in a plant - in particular, in a plant of the species *Beta vulgaris,* more preferably in *Beta vulgaris* subsp. *vulgaris.* In particular, the nucleic acid molecule confers resistance towards a pathogen of the genus Heterodera in a plant in which the polypeptide which is encoded by the nucleic acid molecule is expressed. According to the disclosure, the pathogen is *Heterodera schachtii,* which is among the most important pathogenic nematode of sugar beets and can cause yield loss up to 80%. *Heterodera schachtii* can cause considerable yield loss not only in sugar beets but also in other beets such as red and silver beet, rhubarb and spinach as well as in brassica vegetable crops such as cabbages, Chinese cabbages, cauliflowers, Brussels sprouts, broccoli, turnip, radish and swede, by severely damaging the root system.

The present applicationis based upon the genetic fine mapping, identification, isolation, and characterization of a gene and of a gene locus, respectively, that originates from the donor *Beta vulgaris* subsp. *maritima,* whose presence in a plant - in particular, in *Beta vulgaris* subsp. *vulgaris* - correlates with or is causative for the resistance of the plant concerned to infection with Heterodera. Initial material was a *Beta vulgaris* subsp. *maritima* population collected in France (Hijner 1951).

By means of intensive fine mapping and Map Based Cloning a resistance locus has been identified and sequenced allowing to make sequence comparison between the resistant and the sensitive reference genotype (Fig. 1). The target region was shown to have a high degree of complexity since the resistance locus contains large sequence duplications and in particular in the sensitive genotypes several retrotransposons are embedded in the target region. The resistance locus was found to contain seven annotated genes including three tandemly repeated LRR genes which were identified as candidate genes conferring resistance to Heterodera (LRR1, LRR2 and LRR3), wherein the three LRR genes show sequence-similarity.

Thus, the present application relates to a nucleic acid molecule and to a polypeptide encoded by said nucleic acid molecule, respectively, preferably conferring a resistance towards a pathogen of the genus Heterodera, in particular towards *Heterodera schachtii.* The nucleic acid molecule of the present invention confers - in particular, in a plant of the genus Beta - a resistance to this pathogen. The nucleic acid molecule according to the invention may be an isolated nucleic acid molecule. It is preferably DNA, and, particularly preferably, cDNA (coding DNA). The plant is preferably a plant of the species *Beta vulgaris* - particularly preferably, a plant of the subspecies *Beta vulgaris* subsp. *vulgaris;* among these are, for example, the cultivars sugar beet, beetroot, fodder beet, chard, and Swiss chard.

In one embodiment of the present invention, the nucleic acid molecule according to the invention comprises a nucleotide sequence that comprises the DNA sequence set forth in any one of SEQ ID Nos: 1, 4 and 7 and/or the coding sequence according to any one of SEQ ID Nos: 2, 5 and 8. Furthermore, the present invention provides a nucleotide sequence that encodes a polypeptide with an amino acid sequence according to one of SEQ ID Nos: 3, 6 and 9.

As mentioned above, the gene identified according to the invention is a resistance gene/protein of the type NBS-LRR, which is characterized by specific structural motifs. The general structure of such resistance proteins in plants has already been well-examined (Martin et al., Annual Review Plant Biology 54 (2003), 23-61). However, the principle of the structural embodiment - in particular, of what is known as the LRR domain, which applies as a potential detection domain for most unknown pathogenic effectors - is unpredictable, and the functional background of the resistance genes *i.e.,* the genetic structure, is generally largely unknown. The identification of a Heterodera resistance-conferring gene or protein solely on the basis of the known structural motif is, consequently, impossible. Furthermore, the sequence region has turned out to have a high degree of complexity since the resistance locus contains a large sequence duplication and in sensitive genotypes several retrotransposons are embedded in the target region, which makes the development of diagnostic markers, as well as the assembly of sequence data, especially difficult.

Furthermore, substitutions, deletions, insertions, additions, and/or any other change may be introduced into the DNA sequence of the nucleotide sequence according to the invention that, alone or in combinations, do in fact change the nucleotide sequence, wherein the modified nucleotide sequence may, however, perform the same function as the initial sequence. The present case encompasses a nucleic acid sequence comprising a DNA sequence which is an allele or derivative of the unmodified nucleic acid sequence of the present invention and which when present in the plant confers resistance towards a pathogen of the genus Heterodera, in particular to *Heterodera schachtii.* Furthermore, the present case deals with the coding of an amino acid sequence which confers resistance towards a pathogen of the genus Heterodera, in particular to *Heterodera schachtii.* In a further embodiment, the invention therefore includes a nucleotide sequence that encodes a polypeptide which represents a derivative of the polypeptide which is encoded by the nucleotide sequence according to the invention, or which includes the amino acid sequence according to the invention. A derived amino acid sequence which has at least one substitution, deletion, insertion, or addition of one or more amino acids, wherein the functionality of the encoded polypeptide/protein is preserved, represents a derivative of the polypeptide. Substitutions, deletions, insertions, additions, and/or any other change, either alone or in combinations, that do in fact change the nucleotide sequence, but perform the same function as the initial sequence, may thereby be introduced into the nucleotide sequence using conventional methods that are known in the prior art, *e.g.,* via site-directed mutagenesis, TILLING, PCR-mediated mutagenesis, chemically-induced mutagenesis, genome editing, etc.

The substitution of one amino acid by a different amino acid having the same or equivalent or similar chemical/physical properties is referred to as a "conservative substitution" or "semi-conservative substitution." Examples of physical/chemical properties of an amino acid are, for example, hydrophobia or the charge. Which amino acid substitution represents a conservative or semi-conservative substitution is known to the person skilled in the art. Moreover, general expertise allows the person skilled in the art to recognize, identify, and detect which amino acid deletions and additions are harmless to the functionality of the resistance protein, and at which positions these are possible. The person skilled in the art is aware that, in the case of the present NBS-LRR protein for modifications of the amino acid sequence (substitutions, deletions, insertion, or additions of one or more amino acids), the functionality, in particular, of the conserved domains must be preserved, and that therefore only limited preceding modifications are possible in these domains.

The disclosure thus includes a functional fragment of the nucleotide sequence according to the invention. The term, "fragment," thereby includes genes with a nucleotide sequence sufficiently similar to the aforementioned nucleotide sequence. The term, "sufficiently similar," means that a first nucleotide sequence or amino acid sequence has a sufficient or minimum number of identical or equivalent nucleotides or amino acid groups relative to a second nucleotide sequence or a second amino acid sequence.

With regard to the amino acid sequence, after modification for example via an aforementioned method, this also has a common structural domain and/or possesses common functional activity. Nucleotide sequences or amino acid sequences that have an identity of at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% with the nucleotide sequence or amino acid sequence according to the invention are defined here as being sufficiently similar. This also explicitly encompasses the range of 90% to 100%. For the functional fragments, a sufficient similarity is established if the nucleotide sequence or amino acid sequence generally has the same property as the previously-named nucleotide sequence or amino acid sequence of the present invention. Those nucleotide sequences which encode a derivative or code for a derived amino acid sequence are generated either directly or indirectly (for example, via amplification or replication steps) from an initial nucleotide sequence which corresponds to the nucleotide sequence according to the invention over the entire length, or at least in part.

Accordingly, the present invention includes a nucleotide sequence that is able to hybridize, under stringent conditions, with a nucleotide sequence complementary to a nucleotide sequence according to the invention or to the nucleotide sequence that encodes the amino acid sequence according to the invention and wherein said nucleotide sequence, preferably when present and/or expressed in a plant is capable of conferring resistance towards a nematode of the genus Heterodera.

Furthermore, it is commonly known that the genetic code is redundant, thereby exhibiting a multiplicity of three-base pair codon combinations that specify an amino acid. Thus, the present invention includes a variant DNA sequence due to the degeneracy of the genetic code, which however still preferably when present and/or expressed in a plant is capable of conferring resistance towards a pathogen of the genus Heterodera.

According to the present disclosure, the nucleic acid molecule(s) of the present invention either alone or in combination confer(s) resistance towards a pathogen of the genus Heterodera preferably when present and/or expressed in a plant, more preferably wherein the resistance towards a pathogen of the genus Heterodera is a resistance to *Heterodera schachtii* and/or the plant is a plant of the subspecies *Beta vulgaris* subsp. *vulgaris.*

The nucleic acid molecule according to the invention or the resistance locus of the present invention are characterized in that, preferably when present in a plant and/or upon expression in a plant, they confer a dominant resistance effect against a pathogen of the genus Heterodera - preferably, against *Heterodera schachtii* - or encodes for polypeptides that are able to confer a dominant resistance effect against a pathogen of the genus Heterodera - preferably, against *Heterodera schachtii.*

According to the present invention, a nucleic acid molecule confers resistance towards a pathogen of the genus Heterodera, in particular to *Heterodera schachtii,* preferably when present and/or expressed in a plant, preferably in a plant of the subspecies *Beta vulgaris* subsp. *vulgaris,* wherein the at least two or the three nucleic acid molecules are selected from the group consisting of:
(a) a nucleic acid molecule comprising the nucleotide sequence of the present invention which comprises the DNA sequence set forth in SEQ ID No. 1, the cDNA sequence set forth in SEQ ID No. 2, which hybridizes with a complementary sequence of a nucleotide sequence according to SEQ ID No. 1 or 2, which encodes a polypeptide having the amino acid sequence set forth in SEQ ID No. 3 and/or which is one of the aforementioned alleles, derivatives or variants of the nucleic and amino acid sequence;
(b) a nucleic acid molecule comprising the nucleotide sequence of the present invention which comprises the DNA sequence set forth in SEQ ID No. 4, the cDNA sequence set forth in SEQ ID No. 5, which hybridizes with a complementary sequence of a nucleotide sequence according to SEQ ID No. 4 or 5, which encodes a polypeptide having the amino acid sequence set forth in SEQ ID No. 6 and/or which is one of the aforementioned alleles, derivatives or variants of the nucleic and amino acid sequence;
(c) a nucleic acid molecule comprising the nucleotide sequence of the present invention which comprises the DNA sequence set forth in SEQ ID No. 7, the cDNA sequence set forth in SEQ ID No. 8, which hybridizes with a complementary sequence of a nucleotide sequence according to SEQ ID No. 7 or 8, which encodes a polypeptide having the amino acid sequence set forth in SEQ ID No. 9 and/or which is one of the aforementioned alleles, derivatives or variants of the nucleic and amino acid sequence.

As disclosed, a combination of two nucleic acid molecules described in (a) and (b) confers resistance to Heterodera, in particular to *Heterodera schachtii* when present in a plant, preferably in a plant of the species *Beta vulgaris.*

As disclosed, a combination of the two nucleic acid molecules described in (a) and (c) confers resistance to Heterodera, in particular to *Heterodera schachtii* when present in a plant, preferably in a plant of the species *Beta vulgaris.*

As disclosed, a combination of the two nucleic acid molecules described in (b) and (c) confers resistance to Heterodera, in particular to *Heterodera schachtii* when present in a plant, preferably in a plant of the species *Beta vulgaris.*

The combination may be one or more nucleic acid molecule. However, the combination can also be included in a kit. If the combination is included in a kit the nucleic acids (a) (b) and/or (c) of the combination as described above can be all part of one nucleic acid molecule or can be part of distinct nucleic acid molecules.

**In** this context, the polypeptides/proteins encoded by the combination as defined above are alsodisclosed. The combination of proteins can be comprised in a kit which is also part of the invention.

Disclosed is furthermore a plant which comprises a combination of nucleic acid molecules as described above. The combination can be part of the plant in a transgenic or endogenic way. Furthermore, one or two of the sequences can be part of the plant as transgenes while the other one or two sequences are part of the plants as endogenes.

**In** a further embodiment, the nucleic acid molecule according to the invention is characterized in that, preferably when present in a plant or upon expression in a plant, it already, on its own, confers a dominant resistance effect against a pathogen of the genus Heterodera - preferably, against *Heterodera schachtii* - or that it encodes for a polypeptide that is able to confer a dominant resistance effect against a pathogen of the genus Heterodera.

As disclosed, the nucleic acid molecule as described in (b) in context of combination of inventive nucleic acid molecules confers resistance towards a pathogen of the genus Heterodera, in particular to *Heterodera schachtii,* preferably when present and/or expressed in a plant, in particular in a plant of the subspecies *Beta vulgaris* subsp. *vulgaris.*

As disclosed, the nucleic acid molecule as described in (c) in context of combination of inventive nucleic acid molecules confers resistance towards a pathogen of the genus Heterodera, in particular to *Heterodera schachtii,* preferably when present and/or expressed in a plant, in particular in a plant of the species *Beta vulgaris.*

As disclosed, the nucleic acid molecules described in (a) in context of combination of inventive nucleic acid molecules confers resistance towards a pathogen of the genus Heterodera, in particular to *Heterodera schachtii* when present and/or expressed in a plant, in particular in a plant of the subspecies *Beta vulgaris* subsp. *vulgaris.*

As already described above, until now, Heterodera resistant *Beta vulgaris* subsp. *vulgaris* plants could not be generated without introducing often unwanted features as well, such as, for example, reduced yield, due to the inheritance of additional genes that are linked with the positive feature of Heterodera resistance. A disadvantage of cultivars having the described resistances thus consists in the cultivar development being very laborious and complicated due to the complicated heredity, and in such cultivars having a markedly poorer yield performance relative to normal cultivars, in the absence of an infestation. Among other things, this may be linked to the epigenetic interaction of some resistance genes with genes that are responsible for sugar production, which leads to reduced fitness of the plants, in the absence of the pathogen.

Furthermore, for sustainable breeding against Beet cyst nematode that is to counteract the danger of *Heterodera schachtii* variants that overcome resistance, it is necessary to continuously identify new resistance genes and integrate these into the gene pools of cultivated plants such as sugar beets.

In this context, the inventors for the first time isolated and identified a new Heterodera resistance gene that may be used for markedly simplified breeding. Via the targeted abd facilitated incorporation of this gene in elite lines, it is now possible to very quickly develop very high-yield varieties with a high Heterodera resistance and to provide a further resistance gene which might be used in combating of Heterodera variants that have overcome traditional resistance. Possible accessions for the introgression of one or more of the resistance conferring sequences according to the invention can be acquired for example by screening of populations of *B. vulgaris subsp. maritima.* The screening can rely on the identification of a plant comprising one or more resistance conferring sequences according to the invention. The identification can take place as described elsewhere herein. Preferably the screening or the identification includes the use of molecular markers diagnostic for the resistance locus. Furthermore, plants comprising the resistance conferring sequences can be acquired from CPO Wageningen, Postbus 18, 6700 AA Wageningen/NL, e.g. from accession BMH.

As disclosed, there are provided for the first time a *Beta vulgaris* subsp. *vulgaris* plant like sugar beet plant, a chard plant, a red beet or beetroot plant, a fodder beet plant, having the resistance against a pathogen of the genus Heterodera, in particular against *Heterodera schachtii.* The listed plants are all cultivated plants, crops or plants which are suitable for the agricultural cultivation and which have the resistance. Especially such crops comprise a below-ground storage organ usable as food, raw material or industrial source of sugars or other chemical compound and which comprise the resistance. The storage organ can be for example the beet body of the sugar beet containing sucrose, the consumable beet body of the red beet or the feedable beet body of the fodder beet. The below-ground storage organ can sum up to more than 50% and for the sugar beet even to more than 70% of the total biomass of the full-grown plant. Furthermore, also seeds or seeding material of these plants are disclosed. The seeds or the seeding material can be technically treated as described further below.

The present disclosure includes a nucleic acid that encodes the protein according to any one of SEQ ID Nos. 3, 6 and 9, wherein, in a specific embodiment, the naturally occurring nucleic acid according to any one of SEQ ID Nos. 1, 4 and 7 is excluded.

Furthermore, the present disclosure relates to a recombinant and/or heterologous DNA molecule that comprises the sequences of the nucleic acid molecule according to the invention. This DNA molecule, furthermore, preferably has a regulatory sequence. It may thereby be operatively linked with this regulatory sequence or be under the influence of this regulatory sequence. This regulatory sequence is preferably a promoter sequence and/or other sequences of transcription or translation control elements - for example, cis-elements. The regulatory sequence, which controls the expression of a gene that includes the nucleic acid molecule according to the invention, is preferably a sequence that is able to confer or modulate the expression, as a result of a pathogenic infection. This promoter is preferably able to control the expression of the DNA sequence specifically in roots of the plant. The regulatory sequence may be heterologous to the expressing sequence. Such an approach has the advantage that the person skilled in the art maybetter adjust the expression rate of the sequence to be expressed, the tissue in which the expression occurs, and the point in time at which the expression occurs, in that he selects that regulatory sequence which is best suited to the respective use case. The heterologous DNA sequence preferably includes a nucleotide sequence which encodes a component of the plant pathogen defense (example: resistance genes (R-genes) or genes which encode enzymes involved in signal transfer, such as kinases or phosphatases, and for G-protein, or which encode a pathogenic effector (what are known as avirulence genes *(avr))).* The heterologous DNA sequence may be one of the DNA sequences according to the invention. The heterologous DNA sequence may also additionally encode further components of the plant pathogen defense. The heterologous DNA sequence may therefore be designed such that a polycistronic mRNA is created after its transcription.

The present application furthermore also relates to a polypeptide which can be encoded by the nucleic acid molecule according to the invention and a functionally and/or immunologically active fragment thereof, as well as an antibody that specifically binds to the polypeptide or to its fragment. The polypeptide particularly preferably has an amino acid sequence according to any one of SEQ ID Nos. 3, 6 or 9. The recombinant production of proteins, polypeptides, and fragments is familiar to the person skilled in the art (Sambrook et al., Molecular Cloning: A Laboratory Manual, 3rd ed., Cold Spring Harbor Laboratory Press, Cold Spring Harbor, NY, 2001, or Wingfield, P. T., 2008, Production of Recombinant Proteins, Current Protocols in Protein Science, 52:5.0:5.0.1-5.0.4). Polyclonal or monoclonal antibodies to the protein according to the invention may be produced by the person skilled in the art according to known methods (E. Harlow et al., editor, Antibodies: A Laboratory Manual (1988)). The production of monoclonal antibodies, as well as of Fab and F(ab')₂ fragments that are also useful in protein detection methods, may be performed via various conventional methods (Goding, Monoclonal Antibodies: Principles and Practice, pp. 98-118, New York: Academic Press (1983)). The antibodies may then be used for the screening of expression cDNA libraries in order to identify identical, homologous, or heterologous genes by means of immunological screening (Sambrook et al., Molecular Cloning: A Laboratory Manual, Cold Spring Harbor Laboratory Press, Cold Spring Harbor, NY, 1989, or Ausubel et al., 1994, "Current Protocols in Molecular Biology." John Wiley & Sons), or may be used for western blot analyses. In particular, the present disclosure relates to antibodies that selectively detect a polypeptide encoded by the Heterodera resistance-conferring allele according to the invention, and essentially do not detect the polypeptide encoded by the correspondingly sensitive allele, *i.e.,* that they detect less, by a factor of 2 - preferably, a factor of 5, and, more preferably, a factor or 10 or more - of the polypeptide encoded by the correspondingly sensitive allele than the polypeptide encoded by the Heterodera resistance-conferring allele according to the invention.

As disclosed, the antibody is characterized in that it is a synthetic polypeptide which does not occur in nature.

Furthermore, the antibodies as disclosed may be linked with a fluorescent dye in order to be usable in an immuno-histochemical method, for example, and evoke an antibody coloration. The fluorescent dye may be fluorochrome. The antibodies may also be present linked with other signaling molecules. Among these are, for example, biotin, radioisotopes, reporter enzymes such as alkaline phosphatase, or oligonucleotides.

An additional subject matter of the invention are vectors or expression cassettes that include the nucleic acid molecule or the recombinant DNA molecule according to the invention - possibly under control of regulatory elements and, in particular, under control of functional regulatory elements in plants, as well as negative and/or positive selection markers. The vector backbone is thereby heterologous to the nucleic acid molecule according to the invention, which means that such a vector does not occur in nature and cannot be isolated from nature. The vector is a plasmid, a cosmid, a phage or an expression vector, a transformation vector, shuttle vector or cloning vector; it may be double-stranded or single-stranded, linear or circular; or it may transform a prokaryotic or eukaryotic organism either via integration into its genome or extrachromosomally. The nucleic acid molecule or DNA molecule according to the invention in an expression vector or expression cassette is, preferably, operatively linked with one or more regulatory sequences which allow the transcription and, optionally, the expression in a prokaryotic or eukaryotic cell; (Sambrook et al., Molecular Cloning: A Laboratory Manual, 3rd ed., Cold Spring Harbor Laboratory Press, Cold Spring Harbor, NY, 2001). These regulatory sequences are preferably promoters or terminators - in particular, a transcription initiation starting point, a ribosome binding location, an RNA-processing signal, a transcription termination location, and/or a polyadenylation signal. For example, the nucleic acid molecule is here under the control of a suitable promoter and/or a terminator. Suitable promotors may be constitutive promotors (example: 35S promoter from the "Cauliflower mosaic virus" (Odell et al., Nature 313 (1985), 810 - 812); those promoters which are pathogenically inducible are especially suitable (example: PR1 promoter from parsley (Rushton et al., EMBO J. 15 (1996), 5,690-5,700)). Particularly suitable pathogenically-inducible promoters are synthetic or chimeric promoters which do not occur in nature, are composed of multiple elements, and contain a minimal promoter, and have at least one cis-regulatory element upstream of the minimal promoter, which at least one cis-regulatory element serves as a binding location for special transcription factors. Chimeric promoters are designed according to the desired requirements and are induced or repressed via different factors. Examples of such promoters are found in WO 00/29592, WO 2007/147395, and WO 2013/091612. For example, a suitable terminator is the nos-terminator (Depicker et al., J. Mol. Appl. Genet. 1 (1982), 561-573).

Suitable promoters and terminators may also be the native promoter and the native terminator. The vectors or expression cassettes additionally contain conventional indicator/reporter genes or resistance genes for the detection of the transfer of the desired vector or DNA molecule/nucleic acid molecule, and for selection of the individuals that contain these, since a direct detection via the expression of the gene is for the most part rather difficult. Since the nucleic acid molecule according to the invention here itself encodes for a polypeptide which confers resistance to the beet cyst nematode, it is not essential for the expression in plant cells to provide an additional resistance gene; however, it is recommended, in order to allow a rapid selection.

Examples of indicator/reporter genes are, for example, the luciferase gene and the gene encoding green fluorescent protein (GFP). These, furthermore, also allow tests for the activity and/or regulation of a promoter of the gene. Examples of resistance genes - especially, for plant transformations - are the neomycin phosphotransferase gene, the hygromycin phosphotransferase gene, or the gene encoding phosphinothricin acetyltransferase. Additional positive selection markers may be enzymes which provide the transformed plant a selection advantage over the non-transformed plant - in particular, a nutrient advantage, *e*.*g*., the mannose-6-phosphate isomerase or the xylose isomerase. However, this does not preclude additional indicator/reporter genes or resistance genes known to the person skilled in the art. In a preferred embodiment, the vector is a plant vector. Furthermore, the expression cassette may be present as integrated into a plant genome.

In a further aspect, the present invention relates to cells that include the vectors, recombinant DNA molecules, and/or nucleic acid molecules according to the invention. A cell in the sense of the invention may be a prokaryotic (for example, bacterial) or eukaryotic cell (for example, a plant cell or a yeast cell). The cell is preferably an agrobacterium such as *Agrobacterium tumefaciens or Agrobacterium rhizogenes,* an *Escherichia coli* cell, or a plant cell; the plant cell is particularly preferably a cell of a plant of the genus *Beta,* the species *Beta vulgaris,* or the subspecies *Beta vulgaris* subsp. *vulgaris.* The cell may also be present as a culture. The invention also consequently covers a cell culture which contains such cells. The cell culture is preferably a pure culture or an isolate that contains no cells of another type.

Known to the person skilled in the art are both numerous methods, such as conjugation or electroporation, with which he may introduce the nucleic acid molecule according to the invention, the recombinant DNA molecule, and/or the vector or the expression cassette of the present invention into an agrobacterium, and methods such as diverse transformation methods (biolistic transformation, agrobacterium-mediated transformation) with which he may introduce the nucleic acid molecule according to the invention, the DNA molecule, and/or the vector of the present invention into a plant cell (Sambrook et al., Molecular Cloning: A Laboratory Manual, 3rd ed., Cold Spring Harbor Laboratory Press, Cold Spring Harbor, NY, 2001.

Furthermore, the present disclosure preferably relates to a Heterodera-resistant plant - preferably, a plant of the species *Beta vulgaris* subsp. *vulgaris* or a portion thereof - that contains the nucleic acid molecule according to the invention which confers the Heterodera resistance. The Heterodera-resistant plant may contain the nucleic acid molecule according to the invention as a transgene or as an endogene. According to the disclosure, for the first time, plants of the subspecies *Beta vulgaris* subsp. *vulgaris* were produced which contain the nucleic acid moleculeas described. The disclosure also includes plants of the subspecies *Beta vulgaris* subsp. *vulgaris* which contain the nucleic acid molecule according to the invention as an endogene.

A portion may thereby be a cell, a tissue, an organ, or a combination of multiple cells, tissues, or organs. A combination of multiple organs is, for example, a blossom or a seed. A Heterodera-resistant plant of the present invention preferably shows an enhanced resistance to Heterodera - in particular, *Heterodera schachtii* - than a corresponding plant that does not contain the nucleic acid molecule according to the invention (control plant). The control plant ideally has the identical genotype as the plant of the present invention, and has been cultured under identical conditions, but does not contain the resistance-conferring nucleic acid molecule. The level of the resistance, *e.g.,* to a pathogen of the genus Heterodera, in particular *Heterodera schachtii,* may be qualitatively established in plants of the genus Beta by determining rating scores (see e.g. Example 1). A higher resistance manifests in an improvement in the resistance by at least one rating score, by at least two rating scores, and, preferably, by at least three or more rating scores.

A plant cell or plant or portion thereof that contains the nucleic acid molecule according to the invention - in particular, a plant of the genus Beta - preferably shows a higher resistance to a pathogen of the genus Heterodera - in particular, to *Heterodera schachtii* - than a corresponding plant cell or plant or portion thereof that does not contain the nucleic acid molecule according to the invention, or may contain a sensitive allelic variant of the nucleic acid molecule. The level of the resistance towards a pathogen of the genus Heterodera, *e*.*g*., towards *Heterodera schachtii,* may be qualitatively established in plants of the genus Beta by determining rating scores. A higher resistance manifests in an improvement in the resistance by at least one rating score, by at least two rating scores, and, preferably, by at least three or more rating scores.

**In** the case of a transgenic plant cell, or plant or portion thereof, this comprises the nucleic acid molecule or DNA molecule according to the invention as a transgene or the vector or the expression cassette of the present invention. Such a transgenic plant cell or plant or portion thereof is, for example, one that is transformed - preferably, stably - with the nucleic acid molecule, DNA molecule according to the invention, or with the vector or the expression cassette of the present invention. The nucleic acid molecule is operatively linked with one or more regulatory sequences which allow the transcription and, optionally, the expression in the plant cell. The total structure made up of the nucleic acid molecule according to the invention and the regulatory sequence(s) then represents the transgene. Such regulatory sequences are, for example, a promoter or a terminator. Numerous functional promoters and terminators that are applicable in plants are known to the person skilled in the art.

The disclosure also includes a vacuole of the cell according to the invention, and the content substances (like sucrose) stored therein.

Furthermore, the present application also relates to the cell extract from a cell - preferably, from a plant cell, particularly preferably, from a cell of *Beta vulgaris,* and, especially preferably, from a cell of one of the following crops: sugar beet, chard, or beetroot. No plant can be regenerated from the cell extract. Disclosed herein is a plant genome containing the nucleic acid according to the invention. No plant can be regenerated from the plant genome as such.

The sugar or sucrose concentration from the cell extract may thereby be increased relative to a cell that is not a cell according to the invention, but that belongs to the same species or crop. This applies, in particular, under the conditions when infested by a pathogen of the genus Heterodera.

Also disclosed herein is the use of the cell extract for the production of sugar (sucrose) or for the production of (raw) juice - preferably, beetroot (raw) juice.

Likewise disclosed herein is the sugar - in particular, sucrose - contained in the cells according to the invention and their vacuoles.

An additional aspect of the present disclosure is seed stock comprising seeds that contain the nucleic acid molecule according to the invention. The nucleic acid molecule according to the invention may be present transgenically or endogenously. The seed stock and the seeds may be technically treated. The disclosure thus also comprises technically-treated seed stock and technically-treated seeds. The various kinds of technically-treated seed stock are explained in detail in the following whereby the term seed stock also includes seeds: Technically-treated seed stock may be present in polished form. The outermost layer of the seed is thereby removed, so that the seed assumes a more rounded form. This is helpful in sowing, an optimally uniform shape leads to a uniform distribution of the seed stock grains. Technically-treated seed stock furthermore encompasses pilled seed stock. The seed stock is thereby placed on a pilling mass that protects the seed stock contained therein and leads to a larger mass, such that the pilled seed stock shows a greater resistance capability with regard to wind drift and is thus less susceptible to being blown away by the wind, and, at the same time, a more precise positioning during sowing is enabled. As disclosed, all pilled seed stock grains of a batch or unit designated for sale may have essentially the same shape and the same mass. Deviations of 5% in diameter and mass are possible. However, the deviations preferably do not exceed 1%. As one of the main components, the pilling mass may contain for example a mineral compound such as clay and/or peat, for example. Additional possible components are cited in US 4,067,141. Moreover, the pilling mass may contain additional chemical agents that positively influence the cultivation in practice. These may here be substances that are counted among fertilizing agents. Furthermore, these may be fungicides, insecticides, and/or anti-feedants. The fungicides may be thiram and/or hymexazol and/or other fungicides. The insecticide may be a substance from the neonicotinoid group. The substance from the neonicotinoid group is preferably imidacloprid (ATC Code: QP53AX17) and/or clothianidin (CAS number 210880-92-5). Furthermore, the insecticide may also be cyfluthrin (CAS number 68359-37-5) or beta-cyfluthrin.

The pilled seed stock is a specific kind of dressed seed stock. In this context technically-treated seed stock encompasses also the dressed seed stock. However, the disclosure is not limited to pilled seed stock, but, rather, may be applied with any form of dressed seed stock. The disclosure thus also relates to dressed seed stock, which includes pilled seed stock, but is not limited to this. Dry dressing, wet dressing, and suspension dressing are thus also encompassed. The dressing may thereby also contain at least one dye, such that the dressed seed stock may be quickly differentiated from undressed seed stock, and, furthermore, good visibility in the environment is ensured after sowing. The dressing may also contain those agrochemicals which are described in the context of the pilling mass. The disclosure includes thus such dressed seed stock whereby the dressing contains at least one anti-feedant such as an insecticide and / or at least one fungicide. Optionally, so called electronical dressing (dressing by application of electric energy) may be applied. However, electronic dressing is not a dressing in the strict sense of the word.

An additional form of technically-treated seed stock is encrusted seed stock. What is known as coating is also spoken of in this context as well as of seed stock treated with a coating. The difference to pilled seed stock is that the seed grains retain their original shape, wherein this method is especially economical. The method is described in EP 0 334 258 A1, for example. An additional form of technically-treated seed stock is sprouted or primed seed stock. Sprouted seed stock is pretreated via a pre-germination, whereas primed seed stock has been pretreated via a priming ("germination"). Pre-germinated and primed seed stock have the advantage of a shorter emergence time. The point in time of the emergence after sowing is, at the same time, more strongly synchronized. This enables better agrotechnical processing during cultivation and especially during the harvest, and, additionally, increases the yield quantity. In pre-germination, the seed stock is germinated until the radicle exits the seed stock shell, and the process is subsequently stopped. In the priming, the process is stopped before the radicle exits the seed stock shell. Compared to pre-germinated seed stock, seed stock that has been subjected to a priming is insensitive to the stress of a re-drying and, after such a re-drying, has a longer storage life in comparison to pre-germinated seed stock, for which a re-drying is generally not advised. In this context, technically pre-treated seed stock also includes primed and re-dried seed stock. The process of pre-germination is explained in US 4,905,411 A. Various embodiments of priming are explained in EP 0 686 340 A1. In addition to this, it is also possible to simultaneously pill and prime seed stock in one process. This method is described in EP 2 002 702 B1. Primed seed stock which is moreover pilled, is encompassed by the present invention.

The technically-treated seed stock may additionally be furnished with one or more of the herbicide resistances explained above. This allows a further-improved agrotechnical cultivation, since the technically-treated seed stock may be deployed on a field that has previously been treated with weed killer, and that therefore is weed-free.

In addition to this, the disclosure also encompasses a mixture containing the seed stock or the seeds according to the invention, and a dressing mass as defined above. The dressing mass is thereby preferably embodied as a pilling mass, as defined above.

With storage of seed stock, storage conditions are preferably to be chosen that do not negatively affect the stability or storage life of the seed stock. Fluctuations in humidity may, especially, have a disadvantageous effect here. Also disclosed is a method for the storage of the seed stock in a container that is via simultaneously water-repellent and breathable. Such a container may be designed as a carton. Such a carton may optionally possess an inner vapor barrier. If the carton is designed as a duplex carton, its stability increases. A seed stock according to the disclosure that includes such a container and such a carton, or technically-treated seed stock, is likewise part of thedisclosure . It is likewise part of the disclosure to contain seed stock or technically-treated seed stock in such a carton.

A hybrid plant or a double haploid plant is also disclosed. Hybrid plants and double haploid plants do not occur in nature and cannot be isolated from nature. As disclosed, the nucleic acid molecule is present in heterozygous or homozygous form. In the case of a hybrid plant, the nucleic acid molecule may also be present in hemizygous form. The disclosure also encompasses hybrid seeds and double haploid seeds which contain a nucleic acid according to the invention or a polypeptide according to the invention.

The present disclosure comprises a plant - preferably, of the species *Beta vulgaris* - that is characterized in that the resistance towards a pathogen of the genus Heterodera in this plant is further increased. For example, this may be realized by means of "gene stacking," *i.e.,* the resistance is increased using this dose effect. For this, the plants according to the invention that contain the Heterodera resistance-conferring allele are over-transformed with this resistance allele in order to increase the amount of the transcription of the gene in the plant. An alternative approach includes the gene editing/site-directed mutagenesis or TILLING-mediated modification of the native promoter of the resistance-conferring allele, in order to increase its expression rate, or the modification of the resistance-conferring LRR gene allele itself, in order to increase its activity or stability. Such a method for increasing the activity by means of modification of a resistance gene is described in WO 2006/128444 A2, for example, and may be performed by means of the techniques known to the person skilled in the art. An additional approach may include the fusion of the nucleic acid molecule according to the invention with a heterologous promoter that exhibits a higher activity in comparison to the native promoter - in particular, upon Heterodera infection.

As disclosed, the plant additionally, transgenically or endogenously, may comprise a second nucleic acid molecule at a different position in the genome, which encodes a polypeptide that is able to confer a resistance to Heterodera in the plant in which the polypeptide is expressed. For example, one or more of the resistance genes or resistance loci that are described in the prior art may - insofar as they are not already present in the initial genotype - be introduced into the present plant by means of crossing, transformation, homology-directed repair, or homologous recombination in the plant. Among these are, for example, the Hs1pro-1 gene of *B. procumbens* (Cai et al., Science 275 (1997), 832-834).

The increase in the resistance may take place via integration of the nucleic acid molecule according to the invention into the genome of at least one cell of a plant of the species *Beta vulgaris,* as well as possible regeneration of a plant from the plant cell. The integration may take place both by means of sexual crossing, *e.g.,* with one of the aforementioned *Beta vulgaris* subsp. *maritima* and subsequent selection, or by means of homology-directed repair or homologous recombination. The two latter methods cited are preferably supported by site-directed nucleases which may be selected from, but are not limited to, the following: CRISPR nuclease, including Cas9, CasX, CasY, or Cpf1 nuclease, TALE nuclease, zinc finger nuclease, meganuclease, Argonaut nuclease, restriction endonuclease, including FokI or a variant thereof, recombinase, or two, site-specific, nicking endonucleases.

An alternative approach includes the increase in the expression of the nucleic acid molecule according to the invention in the plant. This may take place via modification of the native promoter, wherein the modification preferably takes place by means of gene editing or site-directed mutagenesis which is mediated via site-directed nucleases, and, optionally, repair models. Examples of such nucleases have already been cited above. The increase in the expression of the nucleic acid molecule according to the invention may likewise take place via fusion of the nucleic acid molecule with a heterologous promoter, which exhibits a higher activity in comparison to the native promoter - in particular, after Heterodera infection. The fusion may likewise take place via site-directed nuclease and repair models, but also by means of direct insertion after double-strand break.

As has already been mentioned above, a method for increasing the Heterodera resistance, may also result in the increase in the activity and/or stability of the polypeptide according to the invention, via modification of the nucleotide sequence of the nucleic acid molecule according to the invention. Such a method for increasing the activity by means of modification of a resistance gene is described in WO 2006/128444 A2, for example, and may be performed by means of the techniques known to the person skilled in the art. This approach is explained in detail further below.

As used herein, a *"site directed nuclease"* (SDN) is an enzyme capable of inducing a double-stranded DNA break at a particular nucleotide sequence, called the *"recognition site".* The SDN can, for example, be selected from the group consisting of meganuclease, TAL effector nuclease, zinc finger nuclease, CRISPR systems like CRISPR/Cas9, CRISPR/Cpf1, CRISPR/CasX or CRISPR/CasY. Rare-cleaving endonucleases are SDN that have a recognition site of preferably about 14 to 70 consecutive nucleotides, and therefore have a very low frequency of cleaving, even in larger genomes such as most plant genomes. Homing endonucleases, also called meganucleases, constitute a family of such rare-cleaving endonucleases. They may be encoded by introns, independent genes or intervening sequences, and present striking structural and functional properties that distinguish them from the more classical restriction enzymes, usually from bacterial restriction-modification Type II systems. Their recognition sites have a general asymmetry which contrast to the characteristic dyad symmetry of most restriction enzyme recognition sites. Several homing endonucleases encoded by introns or inteins have been shown to promote the homing of their respective genetic elements into allelic intronless or inteinless sites. By making a site-specific double strand break in the intronless or inteinless alleles, these nucleases create recombinogenic ends, which engage in a gene conversion process that duplicates the coding sequence and leads to the insertion of an intron or an intervening sequence at the DNA level. A list of other rare cleaving meganucleases and their respective recognition sites is provided in Table I of WO 03/004659 (pages 17 to 20) (incorporated herein by reference).

Furthermore, methods are available to design custom-tailored rare-cleaving endonucleases that recognize basically any target nucleotide sequence of choice. Briefly, chimeric restriction enzymes can be prepared using hybrids between a zinc-finger domain designed to recognize a specific nucleotide sequence and the non-specific DNA-cleavage domain from a natural restriction enzyme, such as Fokl. Such methods have been described e.g. in WO 03/080809, WO 94/18313 or WO 95/09233 and in Isalan et al., 2001, Nature Biotechnology 19, 656- 660; Liu et al. 1997, Proc. Natl. Acad. Sci. USA 94, 5525-5530).

Another example of custom-designed endonucleases includes the so-called TALE nucleases (TALENs), which are based on transcription activator-like effectors (TALEs) from the bacterial genus Xanthomonas fused to the catalytic domain of a nuclease (e.g. FokI or a variant thereof). The DNA binding specificity of these TALEs is defined by repeat-variable diresidues (RVDs) of tandem-arranged 34/35-amino acid repeat units, such that one RVD specifically recognizes one nucleotide in the target DNA. The repeat units can be assembled to recognize basically any target sequences and fused to a catalytic domain of a nuclease create sequence specific endonucleases (see e.g. Boch et al., 2009, Science 326:p1509-1512; Moscou and Bogdanove, 2009, Science 326:p1501; and WO 2010/079430, WO 2011/072246, WO 2011/154393, WO 2011/146121, WO 2012/001527, WO 2012/093833, WO 2012/104729, WO 2012/138927, WO 2012/138939). WO2012/138927 further describes monomeric (compact) TALENs and TALENs with various catalytic domains and combinations thereof.

Recently, a new type of customizable endonuclease system has been described; the so-called CRISPR/Cas system. A CRISPR system in its natural environment describes a molecular complex comprising at least one small and individual non-coding RNA in combination with a Cas nuclease or another CRISPR nuclease like a Cpfl nuclease (Zetsche et al., "Cpf1 Is a Single RNA-Guides Endonuclease of a Class 2 CRISPR-Cas System", Cell, 163, pp. 1-13, October 2015) which can produce a specific DNA double-stranded break. Presently, CRISPR systems are categorized into 2 classes comprising five types of CRISPR systems, the type II system, for instance, using Cas9 as effector and the type V system using Cpfl as effector molecule (Makarova et al., Nature Rev. Microbiol., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova *et al.,* 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems which have been characterized so far. Firstly, the invading DNA, also known as a spacer, is integrated between two adjacent repeat regions at the proximal end of the CRISPR locus. Type II CRISPR systems code for a Cas9 nuclease as key enzyme for the interference step, which system contains both a crRNA and also a trans-activating RNA (tracrRNA) as the guide motif. These hybridize and form double-stranded (ds) RNA regions which are recognized by RNAseIII and can be cleaved in order to form mature crRNAs. These then in turn associate with the Cas molecule in order to direct the nuclease specifically to the target nucleic acid region. Recombinant gRNA molecules can comprise both the variable DNA recognition region and also the Cas interaction region and thus can be specifically designed, independently of the specific target nucleic acid and the desired Cas nuclease. As a further safety mechanism, PAMs (protospacer adjacent motifs) must be present in the target nucleic acid region; these are DNA sequences which follow on directly from the Cas9/RNA complex-recognized DNA. The PAM sequence for the Cas9 from *Streptococcus pyogenes* has been described to be "NGG" or "NAG" (Standard IUPAC nucleotide code) (Jinek et al, "A programmable dual-RNA-guided DNA endonuclease in adaptive bacterial immunity", Science 2012, 337: 816-821). The PAM sequence for Cas9 from *Staphylococcus aureus* is "NNGRRT" or "NNGRR(N)". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpfl nucleases it has been described that the Cpf1-crRNA complex, without a tracrRNA, efficiently recognize and cleave target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized. Further CRISPR effectors like CasX and CasY effectors originally described for bacteria, are meanwhile available and represent further effectors, which can be used for genome engineering purposes (Burstein et al., "New CRISPR-Cas systems from uncultivated microbes", Nature, 2017, 542, 237-241).

The cleavage site of a SDN relates to the exact location on the DNA where the double-stranded DNA break is induced. The cleavage site may or may not be comprised in (overlap with) the recognition site of the SDN and hence it is said that the cleavage site of a SDN is located at or near its recognition site. The recognition site of a SDN enzyme, also sometimes referred to as binding site, is the nucleotide sequence that is (specifically) recognized by the SDN enzyme and determines its binding specificity. For example, a TALEN or ZNF monomer has a recognition site that is determined by their RVD repeats or ZF repeats respectively, whereas its cleavage site is determined by its nuclease domain (e.g. FokI) and is usually located outside the recognition site. In case of dimeric TALENs or ZFNs, the cleavage site is located between the two recognition/binding sites of the respective monomers, this intervening DNA region where cleavage occurs being referred to as the spacer region. In an embodiment of the present invention the recognition site is located in the target region.

A person skilled in the art would be able to either choose a SDN recognizing a certain recognition site and inducing a strand break at a cleavage site at or in the vicinity of the preselected site or engineer such a SDN. Alternatively, a SDN recognition site may be introduced into the target genome using any conventional transformation method or by crossing with an organism having a SDN recognition site in its genome, and any desired DNA may afterwards be introduced at or in the vicinity of the cleavage site of that SDN.

As disclosed, a repair nucleic acid molecule may additionally be introduced into the plant cell.

As used herein, a *"repair matrix"* is a single-stranded or double-stranded DNA molecule or RNA molecule that is used as a template for modification of the genomic DNA at the preselected site in the vicinity of or at the cleavage site. As used herein, *"use as a template for modification of the genomic DNA",* means that the repair matrix is copied or integrated at the preselected site by homologous recombination between the flanking region(s) and the corresponding homology region(s) in the target genome flanking the preselected site, optionally in combination with non-homologous end-joining (NHEJ) at one of the two end of the repair matrix (e.g. in case there is only one flanking region). Integration by homologous recombination will allow precise joining of the repair matrix to the target genome up to the nucleotide level, while NHEJ may result in small insertions/deletions at the junction between the repair matrix and genomic DNA.

A "base editor" as used herein refers to a protein or a fragment thereof having the capacity to mediate a targeted base modification, i.e., the conversion of a base of interest resulting in a point mutation of interest. Preferably, the at least one base editor in the context of the present invention is temporarily or permanently fused to at least one SDN, preferably at least one non-functional SDN, or optionally to a component of at least one (non-functional) SDN. The fusion can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al ((2017). Programmable base editing of A•T to G•C in genomic DNA without DNA cleavage. Nature, 551(7681), 464.) described adenine base editors (ABEs) that mediate the conversion of A•T to G•C in genomic DNA.

As used herein, a modification of the genome means that the genome has changed by at least one nucleotide. This can occur by replacement of at least one nucleotide and/or a deletion of at least one nucleotide and/or an insertion of at least one nucleotide, as long as it results in a total change of at least one nucleotide compared to the nucleotide sequence of the preselected genomic target site before modification, thereby allowing the identification of the modification, e.g. by techniques such as sequencing or PCR analysis and the like, of which the skilled person will be well aware.

As used herein *"a preselected site"* or *"predefined site"* indicates a particular nucleotide sequence in the genome (e.g. the nuclear genome) at which location it is desired to insert, replace and/or delete one or more nucleotides. This can e.g. be an endogenous locus or a particular nucleotide sequence in or linked to a previously introduced foreign DNA or transgene. The preselected site can be a particular nucleotide position at (after) which it is intended to make an insertion of one or more nucleotides. The preselected site can also comprise a sequence of one or more nucleotides which are to be exchanged (replaced) or deleted.

As used herein, a *"flanking region",* is a region of the repair nucleic acid molecule having a nucleotide sequence which is homologous to the nucleotide sequence of the DNA region flanking (i.e. upstream or downstream) of the preselected site. It will be clear that the length and percentage sequence identity of the flanking regions should be chosen such as to enable homologous recombination between said flanking regions and their corresponding DNA region upstream or downstream of the preselected site. The DNA region or regions flanking the preselected site having homology to the flanking DNA region or regions of the repair nucleic acid molecule are also referred to as the homology region or regions in the genomic DNA.

To have sufficient homology for recombination, the flanking DNA regions of the repair nucleic acid molecule may vary in length, and should be at least about 10 nt, about 15 nt or about 20 nt in length. However, the flanking region may be as long as is practically possible (e.g. up to about 100-150 kb such as complete bacterial artificial chromosomes (BACs). Preferably, the flanking region will be about 50 nt to about 2000 nt, e.g. about 100 nt, 200 nt, 500 nt or 1000 nt. Moreover, the regions flanking the DNA of interest need not be identical to the homology regions (the DNA regions flanking the preselected site) and may have between about 80% to about 100% sequence identity, preferably about 95% to about 100% sequence identity with the DNA regions flanking the preselected site. The longer the flanking region, the less stringent the requirement for homology. Furthermore, to achieve exchange of the target DNA sequence at the preselected site without changing the DNA sequence of the adjacent DNA sequences, the flanking DNA sequences should preferably be identical to the upstream and downstream DNA regions flanking the preselected site.

As used herein, *"upstream"* indicates a location on a nucleic acid molecule which is nearer to the 5' end of said nucleic acid molecule. Likewise, the term *"downstream"* refers to a location on a nucleic acid molecule which is nearer to the 3' end of said nucleic acid molecule. For avoidance of doubt, nucleic acid molecules and their sequences are typically represented in their 5' to 3' direction (left to right).

An additional aspect of the present invention is the method for producing a Beta vulgaris plant having resistance towards a nematode of the genus Heterodera as claimed herein.

Herein disclosed is a method for producing a Heterodera-resistant plant, which may take place via transformation of a plant cell with the nucleic acid molecule according to the invention, the recombinant DNA molecule, or with the vector or the expression cassette, and regeneration of the transgenic plant from the transformed plant cell (see Example 3) or via crossing and selection, *e.g.,* with one of the aforementioned *Beta vulgaris* subsp. *maritima.* Vectors or expression cassettes, as well as methods for transforming plants, have already been described above.

The method for production of a Heterodera-resistant plant alternatively includes, as described above, the introduction of a site-directed nuclease and a repair matrix into a cell of a plant, preferably a plant of the species *Beta vulgaris,* wherein the site-directed nuclease is able to generate at least one single-strand break or at least one double-strand break of the DNA in the genome of the cell - preferably, upstream and/or downstream of a target region - and the repair matrix comprises the nucleic acid molecule according to the invention. The method furthermore includes the cultivation of this cell under conditions that allow a homology-directed repair or a homologous recombination, wherein the nucleic acid molecule is incorporated from the repair matrix into the genome of the plant. Furthermore, the regeneration of a plant from the modified plant cell is encompassed.

The target region may be an allelic variant of the nucleic acid molecule according to the invention, wherein the allelic variant when present in a plant does not confer resistance to Heterodera. The allelic variant has been identified to contain retrotransposons.

As described in connection with the nucleic acid molecule according to the invention, substitutions, deletions, insertions, additions, and/or any other change may be introduced that, either alone or in combinations, do in fact change the nucleotide sequence, but perform the same function as the initial sequence - here, the nucleotide sequence of the allelic variant of the nucleic acid molecule according to the invention. Therefore, a nucleotide sequence may represent a derivative of the nucleotide sequence of the allelic variant of the nucleic acid moleculeas disclosed, or an amino acid sequence that represents a derivative of the amino acid sequence of the allelic variant as disclosed. A derived nucleotide or amino acid sequence which has at least one substitution, deletion, insertion, or addition of one or more nucleic acids or amino acids, wherein the functionality of the gene is preserved, represents a derivative of the nucleotide or amino acid sequence. The nucleotide sequence, using conventional methods that are known in the prior art, *e.g.,* via site-directed mutagenesis, TILLING, PCR-mediated mutagenesis, chemical-induced mutagenesis, genome editing, base editing etc., substitutions, deletions, insertions, additions, and/or any other change, either solely or in combinations with the gene, may thereby be introduced, which do in fact change the nucleotide sequence, but perform the same function as the initial sequence.

With regard to the amino acid sequence, after modification via an aforementioned method, this also has a common structural domain and/or possesses common functional activity. Nucleotide sequences or amino acid sequences that at least 80%, at least 85%, at least 90%, at least 91%, at least 92%, at least 93%, at least 94%, at least 95%, at least 96%, at least 97%, at least 98%, at least 99%, or at least 100% identical to the nucleotide sequence or amino acid sequence of the cited allelic variant of the nucleic acid molecule according to the invention are defined here as being sufficiently similar. Accordingly, the present invention includes a nucleotide sequence that is able to hybridize, under stringent conditions, with a nucleotide sequence that is complementary to a nucleotide sequence of the allelic variant of the nucleic acid molecule according to the invention or to the nucleotide sequence that encodes the corresponding amino acid sequence.

Preferably the at least one double-strand break occurs at a position that is at most 10,000 base pairs, preferably at least 5,000 base pairs, more preferably at least 1,000 base pairs, upstream and/or downstream of the target region, or that is at most 10,000 base pairs, preferably at least 5,000 base pairs, more preferably at least 1,000 base pairs, distant from the allelic variantas disclosed.

For the person skilled in the art, it may be obvious that numerous, different sensitive sequences may occur that derive from the nucleic acid molecule according to the invention, but does not confer resistance to Heterodera, such that the sequence listed above should only be considered as an example of sequence. Of course, sensitive variants of the nucleic acid molecule of the present invention may not contain retrotransposons only as described above, but all kinds of mutations known by the person skilled in the art and mentioned above in the DNA or cDNA sequence or in the promoter region may lead to sensitive alleles.

As described above, with quantitative heredity of QTL, not only is the desired resistance towards a pathogen of the genus Heterodera often introduced into the plant, but, rather, also often unwanted features such as, for example, reduced yield, due to the inheritance of additional genes that are not linked with the positive feature of the resistance. Therefore, preferably, the introduction of the nucleic acid molecule according to the invention or the above described combination of nucleic acid molecules, which already shows, on its own, a dominant resistance effect, or of the vector or the expression cassette, is not linked with the introduction of unwanted features, wherein the yield is, preferably, not negatively affected.

Although the QTL analyses which have previously been known from the prior art could detect actual QTL's, the underlying genomic regions that had shown a QTL effect also mediated the disadvantages described above, which is why "linkage drag" is also discussed in this context. At the same time, the QTL's and the effects connected therewith were not described uniformly in the respective prior art, and merely mediated a weak effect, such that the utilization of these results in the breeding of Heterodera-resistant plants was possible to only a limited extent, and was largely uncertain. Targeted breeding and controlled integration of the resistance gene into the gene pool of the sugar beet are now enabled by means of the identification of the resistance gene described herein. This ensures the breeding and generation of entirely new Heterodera-resistant cultivars that exhibit a high resistance to the pathogen, without negatively affecting the sugar yield.

An additional aspect of the present invention is a method for identifying, and optionally providing or selecting, a Beta vulgaris plant that is resistant towards a nematode of the genus Heterodera as claimed herein.

Herein disclosed is a method for the identification, and possibly the provision, of a plant of the species *Beta vulgaris* that is resistant to the pathogen Heterodera, characterized in that the method includes a step of the detection of the presence and/or of the expression of a nucleic acid molecule according to the invention or of the polypeptide according to the invention in the plant or a sample / portion thereof. The presence and/or the expression of a nucleic acid molecule according to the invention, or of the polypeptide according to the invention, may be tested by means of standard methods known to the person skilled in the art, *e.g.,* by means of PCR, RT-PCR, or Western blot.

Furthermore, the identification method as disclosed also includes the detection of the nucleic acid molecule according to the invention by means of detection of at least one polymorphism in the resistance-conferring sequence, *i.e.,* the sequences of the nucleic acid molecule according to the invention. As has already been described above, it may be obvious to the person skilled in the art that numerous sensitive sequences exist, *i.e.,* numerous sequences that encode the allelic variant of the nucleic acid molecule according to the invention. The disclosure consequently includes the detection of at least one polymorphism using molecular markers which detect the polymorphisms - in particular, diagnostic polymorphisms. This detection preferably occurs using at least one molecular marker per polymorphism - in particular, per diagnostic polymorphism. It is known to the person skilled in the art which marker techniques are to be applied to detect a corresponding polymorphism, and how molecular markers for this are constructed (see Advances in Seed Science and Technology Vol. I, Vanangamudi et al., 2008). Furthermore, the present disclosure encompasses molecular markers which describe or detect a polymorphism in the sequences of the nucleic acid molecule according to the invention. It is thereby also possible to use markers that do not differentiate between various polymorphisms, as long as the markers are able to detect such a polymorphism as it occurs in the nucleic acid molecule according to the invention, but is not contained the sensitive allelic variant.

Alternatively or additionally, the identification method as disclosedincludes a step of detecting at least one marker locus in the nucleotide sequence of the nucleic acid molecule according to the invention. As a result of this, a signal is generated, *e.g.,* a fluorescence signal or a sequence amplificate. Furthermore, the preceding identification methods also represent methods for selection of a plant which exhibits the resistance to Heterodera according to the invention. The method for selection includes a concluding step of selecting a resistant plant.

In this context, the present disclosure also includes the development or production of molecular markers that are suitable for detecting the aforementioned polymorphisms of the resistant allele or the construction of hybridization probes that specifically bind to the nucleotide sequence of the nucleic acid molecule according to the invention, or the production of a pair of nucleic acid molecules that is suitable for amplifying, in a PCR, a region that is specific to the nucleic acid molecule according to the invention, and thus for detecting these in a plant or plant cell.

The disclosure preferably includes a method for producing oligonucleotides of at least 15, 16, 17, 18, 19, or 20 - preferably, at least 21, 22, 23, 24, or 25, particularly preferably, at least 30, 35, 40, 45, or 50, and, especially preferably, at least 100, 200, 300, 500 or 1,000 - nucleotides in length that specifically hybridize with a nucleotide sequence of the nucleic acid molecule according to the invention or the nucleic acid molecule that is complementary thereto, or a pair of nucleic acid molecules - preferably, in the form of oligonucleotides - that is suitable for attachment as a forward and reverse primer to a region that is specific to the nucleic acid molecule according to the invention, and for amplifying this in a polymerase chain reaction (PCR), or that is suitable for hybrizidation as a forward and reverse primer to a region in the *Beta vulgaris* genome that, in *Beta vulgaris,* has a co-segregation with the Heterodera resistance conferred by the polypeptide according to the invention or with the nucleic acid molecule according to the invention.

The method for the production of oligonucleotides initially includes: the comparison of the nucleotide sequence of the nucleic acid molecule according to the invention with the nucleotide sequence of the corresponding nucleic acid molecule that does not confer resistance; the identification of the sequence differences between the two nucleotide sequences; and the generation of nucleic acid molecules - here, meaning oligonucleotides - that specifically bind to the nucleic acid molecule according to the invention, but not to the nucleic acid molecule that does not mediate resistance.

Furthermore, the oligonucleotide as disclosed may be connected to a fluorescent dye in order to generate a fluorescence signal, e.g., under excitation via light of the corresponding wavelength. The fluorescent dye may be fluorochrome. The oligonucleotides as disclosed herein may be coupled with other compounds that are suitable for generating a signal. Such oligonucleotides do not occur in nature and also cannot be isolated from nature. The following is executed to produce such marked oligonucleotides: DNA may be marked bio-orthogonally. For this, DNA may be marked *in vivo* or *in vitro* with nucleoside analogs, which, for example, may subsequently be coupled with a fluorophore per Staudinger reaction. In addition to this, DNA may also be chemically provided with fluorophores. Oligonucleotides may be marked via a phosphoramidite synthesis with fluorophores that, for example, are used in QPCR, DNA sequencing, and in situ hybridization. Furthermore, DNA may be generated enzymatically in the course of a polymerase chain reaction with fluorescent nucleotides, or be marked with a ligase or a terminal deoxynucleotidyl transferase. DNA may also be detected indirectly via a biotinylation and fluorescent avidin. For couplings, fluorescein, fluorescent lanthanides, gold nanoparticles, carbon nanotubes, or quantum dots, among other things, are used as fluorophores. One of the most commonly used fluorescent substances is FAM (carboxyfluorescein). Consequently, oligonucleotides and, in particular, primers that possess a FAM marking are encompassed by the invention. FAM is preferably present as 6-FAM, wherein - depending upon the desired wavelength of the emission and excitation - other FAM variants, *e.g.,* 5-FAM, may, however, also be used. Examples of additional fluorescence markers are AlexaFluor, ATTO, Dabcyl, HEX, Rox, TET, Texas Red, and Yakima Yellow. Depending upon the field of use, the oligonucleotides may be furnished with modifications of the bases or of the sugar phosphate spine. Among these are, among others, amino-dT, azide-dT, 2-aminopurine,5-Br-dC, 2'-deoxyinosine (INO), 3'-deoxy-A, C, G, 5-Met-dC, 5-OH-Met-dCN6-Met-dA, and others.

Furthermore, the present disclosure also relates to a marker chip ("DNA chip" or microarray) which contains at least one oligonucleotide as disclosed that is suitable for detection. The marker chip is suitable for application in one or more detection methods as disclosed.

The disclosure likewise includes a method for production of the protein according to the invention. The method includes the provision or cultivation of a cell culture which contains the any one of SEQ ID Nos. 2, 5 and 8, and the subsequent expression of the protein encoded by any one of SEQ ID Nos. 2, 5 and 8.

Furthermore, the present disclosure also relates to a Heterodera-resistant plant or a portion thereof which was identified, and, if applicable, selected, via a method as described in the preceding. In particular, the present disclosure relates to a population of plants comprising plants that are available according to one of the methods as described in the preceding, and that preferably are resistant to the beet cyst nematode, and are characterized by the presence of a nucleic acid molecule according to the invention. The population preferably has at least 10 - preferably, at least 50, more preferably, at least 100, particularly preferably, at least 500, and, particularly in agricultural cultivation, preferably at least 1,000 - plants. The proportion of plants in the population that do not carry the nucleic acid molecule according to the invention and/or are susceptible to infestation with Heterodera, in particular with *Heterodera schachtii* is preferably below 25% - preferably, below 20%, more preferably, below 15%, even more preferably, 10%, and, in particular, preferably below 5%, if present at all.

With the fine mapping described above, the Heterodera resistance-conferring gene(s) in the genome could be identified. This in turn represents the basis for the development of DNA hybridization probes or genetic markers in the target region, with the aid of which the Heterodera resistance-mediating gene could be detected, or could be differentiated from the gene that does not confer resistance.

DNA hybridization probes may be derived from the sequence of the Heterodera resistance-conferring gene(s) and be used for the screening of genomic and/or cDNA banks of the desired organism. The probes may be used to amplify identified homologous genes via the known process of polymerase chain reaction (PCR), and to check whether the Heterodera resistance-conferring gene is present endogenously in an organism, or has been successfully introduced heterologously.

The person skilled in the art may here resort to customary hybridization, cloning, and sequencing methods, which, for example, are listed in Sambrook et al., Molecular Cloning: A Laboratory Manual, 3rd ed., Cold Spring Harbor Laboratory Press, Cold Spring Harbor, NY, 2001. The person skilled in the art may also synthesize and use oligonucleotide primers to amplify sequences of the Heterodera resistance-conferring gene(s). In order to achieve a specific hybridization, such probes should be specific and have at least a length of 15 nucleotides - preferably, at least 20 nucleotides. A detailed guide to hybridization of nucleic acids may be found in Tijssen, Laboratory Techniques in Biochemistry and Molecular Biology - Hybridization with Nucleic Acid Probes, Part 1, Chapter 2, "Overview of principles of hybridization and the strategy of nucleic acid probe assays." Elsevier, New York (1993); and in Current Protocols in Molecular Biology, Chapter 2, Ausubel et al., eds., Greene Publishing and Wiley lnterscience, New York (1995).

Therefore, a nucleic acid molecule of at least 15, 16, 17, 18, 19, or 20 - preferably, at least 21, 22, 23, 24, or 25, particularly preferably, at least 30, 35, 40, 45, or 50, and, especially preferably, at least 100, 200, 300, 500, or 1,000 - nucleotides in length is the subject matter of the presentdisclosure, wherein this nucleic acid molecule specifically hybridizes with a previously-described nucleotide sequence according to the invention that comprises the Heterodera resistance-conferring gene(s). This also explicitly encompasses the range of 15 to 35 nucleotides.

The present disclosure thus also relates to markers as oligonucleotides - in particular, primer oligonucleotides. These comprise a nucleic acid molecule of at least 15 nucleotides in length that specifically hybridizes with a nucleotide sequence defined as in the preceding.

In particular, the present disclosure encompasses a pair of nucleic acid molecules - preferably, in the form of oligonucleotides or a kit containing this pair of oligonucleotides - that is suitable for hybridization as a forward and reverse primer to a region that is specific to the nucleic acid molecule according to the invention, and for amplifying this in a polymerase chain reaction (PCR), or that is suitable as a forward and reverse primer for hybridization to a region in the *Beta vulgaris* genome that, in *Beta vulgaris,* exhibits a co-segregation with the resistance towards a pathogen of the genus Heterodera conferred by the polypeptide according to the invention, or with the nucleic acid molecule according to the invention. Preferably the region in the *Beta vulgaris* genome is located between marker s5e3001s02 and marker s5e4668xxx, is flanked by marker s5e3001s02 and marker s5e4668xxx, or comprises a chromosomal interval between marker s5e3001s02 and marker s5e4668xxx

The following advantages for the breeding and development of new resistant plant lines of the genus Beta may also be achieved via the present invention. Sequence information, as well as the identified polymorphisms which allow a differentiation between resistant and potential susceptible alleles of the disclosed gene, *i*.*e*., between the alleles that confer a resistance towards a pathogen of the genus Heterodera and the alleles that are not capable of conferring this resistance, make possible the marker development which represents an important facilitation for the plant breeder - in particular, with regard to the development of optimized elite lines without "linkage drag." Moreover, knowledge about the sequential structure may be used for the identification of additional resistance genes - in particular, against Heterodera - which are homologous or orthologous, for example.

Therefore, the present disclosure also encompasses a method for the identification of additional nucleic acid molecules which confer a resistance towards a pathogen of the genus Heterodera when present in a plant and which encode polypeptides or additional proteins that are able to confer a resistance towards a pathogen of the genus Heterodera in a plant in which the polypeptide is expressed, respectively. The person skilled in the art may thereby use databases, employing suitable search profiles and computer programs for the screening for homologous sequences or for sequence comparisons. Moreover, by means of conventional molecular biology techniques, the person skilled in the art may himself derive additional DNA sequences encoding Heterodera resistance proteins. For example, suitable hybridization probes may be derived from the sequence of the nucleic acid molecule according to the invention and be used for the screening of genomic and/or cDNA banks of the desired organism. The person skilled in the art may here resort to customary hybridization, cloning, and sequencing methods, which, for example, are listed in Sambrook et al., Molecular Cloning: A Laboratory Manual, 3rd ed., Cold Spring Harbor Laboratory Press, Cold Spring Harbor, NY, 2001. Using known sequences, the person skilled in the art may also synthesize and use oligonucleotide primers to amplify sequences of Heterodera resistance-conferring nucleic acid molecules.

The present disclosure therefore encompasses a method for the identification of a nucleic acid molecule which encodes a polypeptide that is able to confer a resistance to Heterodera in a plant of the species *Beta vulgaris* in which the polypeptide is expressed. The method thereby includes the comparison of the amino acid sequence of the polypeptide according to the invention which, in *Beta vulgaris* subsp. *vulgaris,* confers a resistance towards a pathogen of the genus Heterodera with amino acid sequences from a sequence database, or with sequences of allelic variants of the polypeptide according to the invention in genotypes of the species *Beta vulgaris.* Furthermore, the method as disclosed includes the identification of an amino acid sequence or of an allelic variant that is at least 70%, preferably at least 80% identical to the amino acid sequence of the polypeptide according to the invention, as well as the introduction of a nucleic acid molecule encoding the identified amino acid sequence or allelic variant in a plant of the species *Beta vulgaris;* expression of the nucleic acid molecule in the plant; and, optionally, subsequent verification of the resistance towards a pathogen of the genus Heterodera.

As described in the preceding, additional Heterodera resistance-conferring proteins or their coding genes, *i*.*e*., homologs, analogs, and orthologs, that are at least 70% - preferably, at least 80%, particularly preferably, at least 90%, especially preferably, at least 95%, or even 98% - identical to the amino acid sequence of the polypeptide which is encoded by the nucleic acid molecule according to the invention may be identified via classical bioinformatic approaches (database searches and computer programs for screening for homologous sequences).

The disclosure further includes a plant or a seed comprising the nucleid acid of the invention as described herein wherein the plant or the seed of such a plant has a genome allowing the development of a beet body having a minimum fresh mass of 200g, 250g, 300g, 350g, 400g, 450g or 500g and a maximum mass of 100g, 1100g, 1200g, 1300g, 1400g, 1500g, 1600g, 1700g, 1800g, 1900g or 2000g. Corresponding genetic composition for the development of such a beet body is available for example via the following varieties BTS 8629, BTS 8735, BTS 8500, BTS 8767 or BTS 8749. The person skilled in the art knows how to transfer the nucleic acid according to the invention into a plant having such a genetic composition. The seed in this embodiment may be a pelleted seed.

The disclosure further includes a plant or a seed comprising the nucleid acid of the invention as described herein wherein the plant or the seed of such a plant has a genome allowing the development of a beet body having a saccharose concentration in the fresh mass of the beet body of at least 10%, 11%, 12%, 13%, 14%, 15%, 16%, 17%, 18%,19% or even 20% (percent by mass).

The term, homolog(s), thereby means that the genes concerned (from two different plant species) have essentially the same function and a common ancestor, and therefore typically show a significant identity in their nucleic acid or coded amino acid sequences. However, there are also many genes that are homologous to one another, without protein sequences resulting in a meaningful paired alignment. In contrast to this, the term, analog(s), describes genes or proteins that (likewise) have an identical or similar function, but are not created from the same structure, *i.e.,* have no common ancestor. In this case, often, no significant identity can be established in their nucleic acid or encoded amino acid sequence, or, in the best case, in specific functional domains.

In the context of genome sequencing, homologs are, for annotation, more finely classified. The terms, orthology and paralogy, have been introduced for this. Orthologs are genes that are connected via a speciation event. Paralogs are genes that trace back to a duplication event.

A gene is, then, fundamentally a homolog or analog or ortholog in the sense of the present invention if it is able to confer Heterodera resistance in a plant. To check, methods, which have already been described in the preceding, known to the person skilled in the art are used, *e.g.,* the amplification of the identified homolog or analog or ortholog by means of PCR, cloning in expression vectors, introduction into the target plant or plant cell, and checking the resistance.

As described above, the usage disclosed here of the resistant gene allele in cis- or transgenetical approaches opens up the possibility of new resistant species of the genus Beta which, using the dose effect, exhibit a enhanced resistance, or in which a resistance break may be avoided and the resistance development optimized via the stacking of the disclosed gene with other resistance genes. Modifications of the gene by means of tilling or targeted engineering to develop new resistance alleles are also possible.

The present disclosure also relates to the use in a plant of the identified Heterodera resistance-conferring gene allele in a genetic or molecular stack with other genetic elements which may confer agronomically advantageous properties. The economic value of cultivated plants may thereby be markedly increased, in that, for example, the yield performance is increased in comparison to plants that possess the same genetics, but have not been furnished with the nucleic acid according to the invention. Furthermore, new crop areas for a plant may be opened up that were not previously accessible to the cultivation of this plant due to biotic factors such as strong pathogen pressure. In particular, the present disclosure relates to the use of the identified Heterodera resistance-conferring gene allele in methods for controlling an infestation with the pathogen *Heterodera schachtii* in the agricultural or horticultural cultivation of plants of the genus Beta, *e*.*g*., encompassing the identification and selection of plants of the genus Beta with the aid of one of the methods described in the preceding and/or the cultivation of the plants so selected or descendants thereof. The present disclosure thus includes a method for the cultivation of plants of the species *Beta vulgaris,* including, in a first step, the provision of Heterodera-resistant plants of the species *Beta vulgaris* as disclosed, or the production of plants of the species *Beta vulgaris* with the aid of the production methodas disclosed, or the identification and selection of plants of the species *Beta vulgaris* with the aid of the identification method according to the invention that has been described in the preceding; and including, in a second step, the cultivation of the plants from the first step, or the deployment of seed stock of the plants from the first step, or the raising of plants from the first step. The cultivation method thereby counteracts an infestation of the cultivated plants by Heterodera. The cultivation method may be part of a method for producing sugar. The method for the production of sugar includes the steps of the cultivation method, and additionally, as a penultimate step, the harvesting of the cultivated plants, and, as a last step, the extraction of sugar from the aforesaid plants.

The cultivation method may also be part of a method for producing seed stock. The method for the production of seed stock includes the steps of the cultivation method, and additionally, as a penultimate step, the vernalization of the cultivated plants, and, as a last step, the extraction of seeds from the aforesaid plants. The extracted seeds may optionally be pilled, in order to obtain pilled seed stock of the species *Beta vulgaris.* In this instance, it is a method for the production of pilled seed stock.

Moreover, the method for the production of seed stock may be designed as a method for the production of Heterodera-resistant seed stock. The method for the production of Heterodera-resistant seed stock includes the steps of the method described above for the production of seed stock, and additionally, as a last step, the verification of the nucleic acid according to the invention according to a method described herein in at least one of the extracted seeds - preferably, in at least 0.1% or in at least 1% of the extracted seeds. The verification is particularly preferably implemented so that the seed remains germinable. This means that the extraction of the DNA required for verification from the seed does not neutralize the germinability of the seed. In such an instance, the verification of the nucleic acid according to the invention may have taken place in an especially large proportion of all extracted seeds. For example, the verification may take place in at least 2% - preferably, at least 3%, particularly preferably, at least 4% - of all extracted seeds.

The plants as disclosed, their cells, or seeds according to the invention may possess additional, agronomically advantageous properties, or be furnished with such. One example is the tolerance or resistance to an herbicide such as glyphosate, glufosinate, or ALS inhibitors. The tolerance to glyphosate or an ALS-inhibitor herbicide is preferred. A specific embodiment of the glyphosate resistance is disclosed in US 7,335,816 B2. Such a glyphosate resistance is, for example, available from seed stock stored at the NCIMB, Aberdeen (Scotland, UK), under the access number, NCIMB 41158 or NCIMB 41159. Such seeds may be used in order to obtain a glyphosate-tolerant sugar beet plant. The glyphosate resistance may also be introduced into other species of the genus *Beta* via crossing.

The disclosure thus also encompasses plants, their cells, or seeds or seed stock, characterized in that these contain the nucleic acid molecule according to the invention, and furthermore in that a DNA fragment of the genomic DNA of the plant, portions, or seeds thereof may be amplified via polymerase chain reaction with a first and a second primer.

A specific embodiment of the ALS-inhibitor herbicide resistance is disclosed in the document, WO 2012/049268 A1. For example, such an ALS-inhibitor herbicide resistance is available from a deposit of NCIMB, Aberdeen, UK, under the number NCIMB 41705. Furthermore, such an ALS-inhibitor resistance may be produced via tilling or site-directed mutagenesis, *e*.*g*., via gene editing, such as through the use of CRISPR/Cas. The invention thus also encompasses plants, their cells, or seeds or seed stock, characterized in that these contain the nucleic acid molecule according to the invention, and furthermore in that these exhibit a mutation in an endogenous acetolactate synthase gene, wherein the acetolactate synthase gene encodes an acetolactate synthase protein which, as a result of the mutation at position 569, has a different amino acid than tryptophan. As a result of the mutation, the amino acid at position 569 is preferably alanine, glycine, isoleucine, leucine, methionine, phenylalanine, proline, valine, or arginine. Furthermore, the mutation may be present both heterozygously and homozygously in the plants, their cells or seeds, or the seed stock. We recommend the homozygous presence of the mutation, since this promotes a more stable or more intensive phenotypical occurrence of the resistance.

Numerous additional herbicides and their applicability are known to the person skilled in the art from the prior art. He may resort to the prior art in order to achieve knowledge of which genetic elements are to be used in what manner in order to implement a corresponding tolerance in plants.

A further example of an agronomically advantageous property is an additional pathogen resistance, wherein pathogens may be insects, viruses, nematodes, bacteria, or fungi, for example. For example, a broad pathogen defense for a plant may be achieved via combination of different pathogen resistances/tolerances, since genetic elements may exhibit additive effects among one another. For example, numerous resistance genes for this are known to the person skilled in the art as genetic elements. For example, US 2016/0152999 A1 discloses an RZ resistance gene against the disease Rhizomania. This disease is caused by the pathogen, "Beet Necrotic Yellow Vein Virus." Several disease resistances contained in one plant have synergistic effects upon one another. If a plant is infested for the first time by a pathogen, its immune system is normally weakened, and the epidermis as an outer barrier is often damaged, such that the probability of further infections is increased. An additional example of an agronomically advantageous property is cold tolerance or frost tolerance. Plants which exhibit this property may already be sown earlier in the year, or may remain in the field longer, which may lead to increased yields, for example. Here, the person skilled in the art may also resort to the prior art to find suitable genetic elements. Additional examples of agronomically advantageous properties are water usage efficiency, nitrogen usage efficiency, and yield. Genetic elements which may be used to confer such properties might be found in the prior art.

Furthermore, numerous modifications for pathogen defense are known to the person skilled in the art. In addition to the families of the R-genes that are often described, the Avr/R approach, the Avr gene complementation (WO 2013/127379), the autoactivation of an R-gene (WO 2006/128444), or the HIGS (host-induced gene silencing) approach (e.g., WO 2013/050024) may be advantageously used. In particular, the autoactivation of an R-gene might be important to the present invention. For this, a nucleic acid is to be created that encodes an autoactivated resistance protein for generation of a resistance to pathogens in plants. This nucleic acid then has only a limited portion of an NBS-LRR resistance gene, such as the wb-R-gene, which extends downstream from the 5' end of the coding region of the NBS-LRR resistance gene to the beginning of the coding for the NBS domain of the NBS-LRR resistance gene.

In this context, a method is also disclosed herein which contains the step of the removal of that region of the nucleic acid according to the invention which encodes the N-terminal region and which begins with the p-loop in the NBS domain, and extends up to the end of the N-terminal region.

The resistance proteins that are encoded for by such shortened nucleic acids are generally autoactivated, in that these resistance proteins trigger an immune reaction in the plant, even in the absence of the associated pathogen, and thus increase the base immunity of the plant. Furthermore, such a shortened nucleic acid according to the invention, and the polypeptide that is encoded by this, are disclosed.

Furthermore, the disclosure also includes the use of the Heterodera resistance-conferring gene allele, identified with a method described above, for combination with one of the preceding modifications, or with a genetic element described in the preceding which may convey in a plant one or more agronomically advantageous properties.

In addition to the plant, the present disclosure also relates to seeds or descendants, or to an organ, a plant part, a tissue, or a cell thereof in the production of products that are typically produced from sustainable raw materials, such as foodstuffs and animal feed - preferably, sugar or syrup (molasses), wherein the molasses is also used for industrial applications, *e.g.,* in alcohol production or as a growing medium for the production of biotechnological products, in the production of materials or substances for the chemical industry, *e.g.,* refined chemicals, pharmaceuticals or precursors thereof, diagnostics, cosmetics, bioethanol, or biogas. An example of the use of sugar beet as a biogenic raw material in biogas plants is described in the application DE 10 2012 022 178 A1; see, for example, paragraph 10.

The following examples explain the invention. Unless indicated otherwise, standard molecular biology methods have been used; see, for example, Sambrook et al., Molecular Cloning: A Laboratory Manual, 3rd ed., Cold Spring Harbor Laboratory Press, Cold Spring Harbor, NY, 2001, Fritsch *et al.,* Cold Spring Harbor Laboratory Press: 1989; Mayer et al., Immunochemical Methods in Cell and Molecular Biology, eds., Academic Press, London, 1987, and Weir et al., Handbook of Experimental Immunology, Volumes I-IV, Blackwell, eds., 1986.

Some of the most important sequences according to the invention are explained in detail in the following:
- SEQ ID No. 1: genomic DNA sequence of the Heterodera resistance-conferring LLR2 gene from *Beta vulgaris subsp. maritima.*
- SEQ ID No. 2: cDNA sequence of the Heterodera resistance-conferring LLR2 gene as it does not occur in nature.
- SEQ ID No. 3: amino acid sequence of the Heterodera resistance-conferring LLR2 protein as it is encoded by SEQ ID No. 1 or SEQ ID No. 2.
- SEQ ID No. 4: genomic DNA sequence of the Heterodera resistance-conferring LLR1 gene from *Beta vulgaris subsp. maritima.*
- SEQ ID No. 5: cDNA sequence of the Heterodera resistance-conferring LLR1 gene as it does not occur in nature.
- SEQ ID No. 6: amino acid sequence of the Heterodera resistance-conferring LLR1 protein as it is encoded by SEQ ID No. 4 or SEQ ID No. 5.
- SEQ ID No. 7: genomic DNA sequence of the Heterodera resistance-conferring LLR3 gene from *Beta vulgaris subsp. maritima.*
- SEQ ID No. 8: cDNA sequence of the Heterodera resistance-conferring LLR3 gene as it does not occur in nature.
- SEQ ID No. 9: amino acid sequence of the Heterodera resistance-conferring LLR3 protein as it is encoded by SEQ ID No. 7 or SEQ ID No. 8.
- SEQ ID No. 10: molecular marker s5e3001s02
- SEQ ID No. 11: molecular marker s5e4668xxx

### EXAMPLES

### Example 1: Carrying out of a Nematode test

1) Plants according to the invention were sown in greenhouse peat substrate
2) The plants were transplanted after emergence in the cotyledon stage in ca. 30 ml (ca. 2*1*15 cm) plastic boxes filled with quartz sand as single plants (one plant/box). Alternatively, plantlets from tissue culture can be transferred directly in the plastic boxes. Temperature and light conditions: 16/8 hours light/dark with 23°C/12°C temperature change.
3) One week after transplanting infestation was simulated by application of 600 larvae of *Heterodera schachtii.*
4) Evaluation of plants and number of cysts was done 4 weeks after infection under the binocular.

### Example 2: Assembling of genes

The resistance locus is located on chromosome 5 and the target region was reduced in several mapping steps to the flanking markers s5e5864s01 (Integrated genetic map: 9,17 cM) and s5e4503s02 (9,74 cM) enclosing a physical distance of 119341 bp in the reference physical map (ZR_BPMv7 - monogerm sensitive reference sequence). In a BAC screening of the resistant donor line, 3 BAC clones for the target genomic region were identified. The BAC clones were sequenced by use of the PacBio method (Fichot, Erin B., and R. Sean Norman. "Microbial phylogenetic profiling with the Pacific Biosciences sequencing platform." Microbiome 1.1 (2013): 10). This method allows to produce longer contigues sequence contigs and thereby fascillitates the subsequent assembling of contigs generated from genomic areals containing repetitive sequences. A gapless resistant sequence was assembled allowing to make sequence comparisons between the resistant and the sensitive reference genotype and to develop new markers in the target region. In a new fine mapping step using the available recombinants the target region was reduced to two new flanking markers enclose a sequence stretch of 26484 bp in the resistant and 39587 bp in the sensitive genotype. The reduced target region contains only 9 annotated genes. Among these genes, 3 tandemly repeated LRR genes were identified as potential causal candidate genes for the NRBMH resistance (LRR1, LRR2 and LRR3 (Figure 1)). The target region shows a high degree of complexity: a) the resistant sequence contains a large sequence duplication; b) the LRR genes are showing sequence-similarity; c) in sensitive genotypes several retrotransposons are embedded in the target region. Because of the sequence complexity, the assembly of the RR and ss sequences was a highly demanding and very complex procedure.

Within the reduced targeted region, 5 recombinations were detected and 180 descendants of these recombinants were phenotyped. The phenotypes were determined with intensive statistical methods (t-Test, Power analysis). From these 5 recombinants, a sample of 10 plants per Ident were analyzed with specific dominant markers developed for the 3 LRR genes. It was possible to test the 3 LRR genes for functionality. As an exemplary result, the LRR1 gene shows in none of the recombinants any conflicting data, i.e. all recombinants carrying the LRR1 gene are resistant.

The "Map Based Cloning" process involved the following steps: Genetic fine mapping, physical mapping, WHG (whole genome) sequence analysis, construction of several large segregating populations, recombinant screen, marker development in the target region, comparative BAC sequencing in the resistant genotype, analysis of resistant and sensitive genotype sequences, bioinformatic analyzes, protein prediction, comparison of proteins. The following steps were crucial for this invention: fine mapping coupled with intensive phenotyping, identification and sequencing of resistant BAC clones, development of dominant markers for the 3 LRR genes, sequence analysis and sequence and protein comparisons between RR (resistant) and ss (susceptible) genotypes.

### Example 3: Introduction of the resistance-conferring gene as a transgene by means of gene transformation in Beta vulgaris subsp. vulgaris

The transgenic approach to the production of *Heterodera*-resistant plants served not only for the alternative validation of the LRR gene(s) as the resistance-conferring gene(s), but also as a means of producing transgenic resistance events that confers a novel *Heterodera* resistance or improve already existing *Heterodera* resistances.

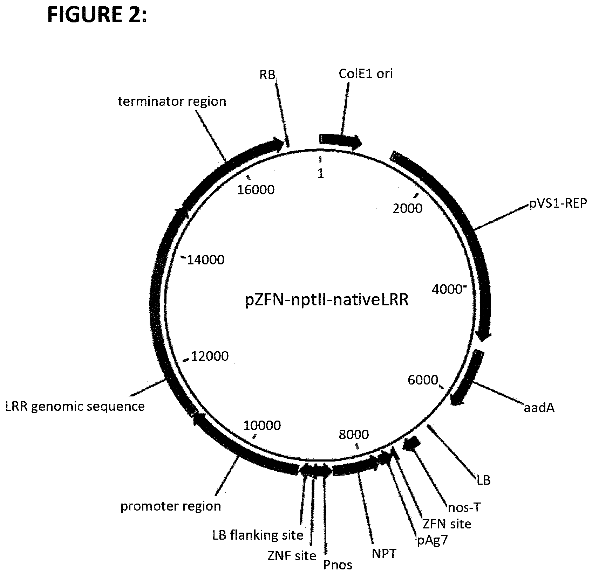

The LRR gene of interest was cloned into the binary vector pZFN-nptII (Figure 2) by means of the following standard cloning procedures: Within the T-DNA of this vector, the cDNA of the resistance gene was cloned between the duplicated CaMV 35S promoter and the nopaline synthase (NOS) terminator, in order to ensure a high, constitutive expression level of the resistance gene in the transgenic plant. The T-DNA furthermore includes the neomycin phosphotransferase II (*nptII*) gene, which confers resistance to a bandwidth of aminoglycoside antibiotics such as kanamycin or paromomycin. These antibiotic resistances were used for the selection of the transgenic plant cells and tissues. The *NOS* promoter and the *pAG7* terminator flank the *nptII* gene. The backbone of the binary vector furthermore contains the *colE1* and the *pVS1* origin for the plasmid replication in *Escherichia coli* or *Agrobacterium tumefaciens.* The *aadA* gene confers streptomycin / spectinomycin resistance for bacteria selection. The pZFN-nptII-LRR plasmid was transformed in agrobacterium strain AGL-1 by means of standard procedure.

The transformation of the sugar beets took place according to Lindsey & Gallois (1990), "Transformation of sugarbeet (Beta vulgaris) by Agrobacterium tumefaciens." Journal of experimental botany 41.5, 529-536.). For this, "micropropagated shoots" of genotype 04E05B1DH5, which carries exclusively sensitive alleles of the identified gene, were used as starting material. Shoots were multiplied in the corresponding medium according to Lindsey & Gallois (1990). In order to induce as many meristems as possible, the "shoots" were transferred into a different medium (see Lindsey & Gallois (1990)) and incubated in darkness for several weeks at approximately 30 °C. Agrobacterium strain AGL-1 with vector pZFN-nptII-LRR was cultured in an additional medium (see Lindsey & Gallois (1990)), additionally provided with corresponding antibiotics for selection. Sections of meristemic tissue based upon the shoot to be treated were incubated with agrobacterium for several hours in an additional medium (see Lindsey & Gallois (1990)). Plant explants and agrobacteria were co-cultivated in darkness for at least 2 days in medium (see Lindsey & Gallois (1990)), and inoculated explants were subsequently incubated in darkness for approximately 2 weeks in an additional medium (see Lindsey & Gallois (1990)). The explants were thereupon further propagated in an additional medium (see Lindsey & Gallois (1990)) and sub-cultivated, in order to enable the selection of the transgenic tissue. Leaf material was then extracted from the green, growing "shoots" and examined by means of PCR for the presence of the transgene. Suitable "shoots" were rooted and subsequently transferred to a greenhouse for production of T1 seed stock. Heterodera resistance in T1 plants can then be tested by means of the protocol describe din Example 1.

## Claims

1. A nucleic acid molecule for increasing the resistance towards a nematode of the genus Heterodera in a Beta vulgaris plant in which the nucleic acid molecule is expressed thereby characterized that the nucleic acid molecule is selected from the group consisting of:
(a) a nucleotide sequence which comprises the sequence selected from the group consisting of: SEQ ID NO 1, 4 and 7;
(b) a nucleotide sequence which comprises the coding sequence selected from the group consisting of: SEQ ID NO 2, 5 and 8;
(c) a nucleotide sequence which hybridizes with a complementary sequence of a nucleotide sequence according to (a), (b), (f) or (g) under stringent conditions;
(d) a nucleotide sequence which comprises a sequence that is at least 90% identical to the sequence of the nucleotide sequence of any one of (a) or (b);
(e) a nucleotide sequence encoding a polypeptide having an amino acid sequence selected from the group consisting of: SEQ ID NO 3, 6 and 9;
(f) a nucleotide sequence encoding a polypeptide having an amino acid sequence that is at least 94% identical to the amino acid sequence selected from the group consisting of: SEQ ID NO 3, 6 and 9;
(g) a nucleotide sequence which is a variant of a DNA sequence of any of (a) to (f) due to the degeneracy of the genetic code,
wherein stringent conditions are defined as hybridization in 4 x SSC at 65°C, and subsequent repeated washing in 0.1 x SSC at 65°C for approximately 1 hour in total.

2. Polypeptide which is encoded by the nucleic acid molecule according to claim 1.

3. Vector or expression cassette comprising the nucleic acid molecule according to claim 1.

4. Cell which comprises the nucleic acid molecule according to claim 1 as a transgene, or the vector or expression cassette according to claim 3.

5. Seed of a Beta vulgaris plant, **characterized in that** the plant is not *B. vulgaris* subsp. *maritima,* wherein the seed comprises the nucleic acid molecule according to claim 1, wherein the seed is not exclusively obtained by means of an essentially biological process.

6. The seed of a Beta vulgaris plant according to claim 5, wherein the seed has been technically treated, whereby the technical treatment is selected from the group consisting of:
(a) Polishing
(b) Dressing preferably pelleting
(c) Incrustation
(d) Colouring.

7. Method for increasing the resistance to a nematode of the genus Heterodera in a Beta vulgaris plant, including the following steps:
(i) integration of the nucleic acid molecule according to claim 1 by means of homology-directed repair into the genome of at least one cell of a plant, and regeneration of a plant from the plant cell; or
(ii) increase in the expression of the nucleic acid molecule according to claim 1 in the plant by modification of the native promoter or by fusion of the nucleic acid molecule with a heterologous promoter that exhibits a higher activity in comparison to the native promoter; or
(iii) transformation of a plant cell with the nucleic acid molecule according to claim 1, or the vector or the expression cassette according to claim 3, and regeneration of the transgenic plant from the transformed plant cell.

8. Method for increasing the resistance to a nematode of the genus Heterodera in a Beta vulgaris plant according to claim 7, wherein homology-directed repair is promoted by a site-directed nuclease.

9. Method for producing a Beta vulgaris plant having resistance towards a nematode of the genus Heterodera, including the following steps:
(a) transformation of a plant cell with the nucleic acid molecule according to claim 1, or the vector or the expression cassette according to claim 3; and
(b) regeneration of the transgenic plant from the transformed plant cell; or
(i) introduction of a site-directed nuclease and a repair matrix into a cell of a plant, wherein the site-directed nuclease is able to generate at least one single-strand break or at least one double-strand break of the DNA in the genome of the cell upstream and/or downstream of a target region and the repair matrix comprises the nucleic acid molecule according to claim 1;
(ii) cultivation of the cell from (i) under conditions that allow a homology-directed repair or a homologous recombination, wherein the nucleic acid molecule is integrated from the repair matrix into the genome of the plant; and
(iii) regeneration of a plant from the cell modified in (ii),

10. Method according to claim 9, **characterized in that** the target region
a) is located between a marker according to SEQ ID NO: 10 and a marker according to SEQ ID NO: 11, or
b) is flanked by a marker according to SEQ ID NO: 10 and a marker according to SEQ ID NO: 11, or
c) comprises a chromosomal interval between a marker according to SEQ ID NO: 10 and a marker according to SEQ ID NO: 11.

11. Method according to claim 9 or 10, **characterized in that** the at least one single-strand break or at least one double-strand break occurs at a position that is at most 10,000 base pairs upstream and/or downstream of the target region.

12. Method for identifying, and selecting, a Beta vulgaris plant that is resistant towards a nematode of the genus Heterodera, **characterized in that** the method includes at least step (i) or (ii):
(i) detection of the presence and/or expression of the nucleic acid molecule according to claim 1, or the presence of the polypeptide according to claim 2, in the Beta vulgaris plant or a portion of the Beta vulgaris plant; and/or
(ii) detection of at least one region which is located between the marker according to SEQ ID NO 10 and the marker according to SEQ ID NO 11, is flanked by the marker according to SEQ ID NO 10 and the marker according to SEQ ID NO 11, or comprises a chromosomal interval between the marker according to SEQ ID NO 10 and the marker according to SEQ ID NO 11, co-segregating with the nucleotide sequence of the nucleic acid molecule according to claim 1; and
(iii) selection of the Beta vulgaris plant having resistance towards a nematode of the genus Heterodera,
wherein the marker according to SEQ ID NO 10 is a single nucleotide polymorphism (SNP) at position 56940072 bp of chromosome 5 referenced to Beta vulgaris genotype EL10, wherein said nucleotide is T, and the marker according to SEQ ID NO 11 is a single nucleotide polymorphism (SNP) at position 57809807 bp of chromosome 5 referenced to Beta vulgaris genotype EL10, wherein said nucleotide is T.

13. Use of an oligonucleotide primer of at least 20 nucleotides in length, which oligonucleotide specifically hybridizes as probe with any of the nucleotide sequences selected from the group consisting of
(i) a nucleotide sequence which comprises the sequence selected from the group consisting of: SEQ ID NO 1, 4 and 7;
(ii) a nucleotide sequence which comprises the coding sequence selected from the group consisting of: SEQ ID NO 2, 5 and 8;
(iii) a nucleotide sequence encoding a polypeptide having an amino acid sequence selected from the group consisting of: SEQ ID NO 3, 6 and 9,
for amplifying of a region specific to the nucleic acid molecule of claim 1 in a polymerase chain reaction.

## Patentansprüche

1. Nukleinsäuremolekül zur Erhöhung der Resistenz gegen einen Nematoden der Gattung Heterodera in einer *Beta vulgaris*-Pflanze, in der das Nukleinsäuremolekül exprimiert wird, **dadurch gekennzeichnet, dass** das Nukleinsäuremolekül ausgewählt ist aus der Gruppe bestehend aus:
(a) einer Nukleotidsequenz, die die Sequenz umfasst, die aus der Gruppe bestehend aus SEQ ID Nr. 1, 4 und 7 ausgewählt wurde;
(b) einer Nukleotidsequenz, die die kodierende Sequenz umfasst, die aus der Gruppe bestehend aus SEQ ID Nr. 2, 5 und 8 ausgewählt wurde;
(c) einer Nukleotidsequenz, die mit einer komplementären Sequenz einer Nukleotidsequenz gemäß (a), (b), (f) oder (g) unter stringenten Bedingungen hybridisiert;
(d) einer Nukleotidsequenz, die eine Sequenz umfasst, die zu mindestens 90% mit der Sequenz der Nukleotidsequenz gemäß (a) oder (b) identisch ist;
(e) einer Nukleotidsequenz, die ein Polypeptid kodiert mit einer Aminosäuresequenz, ausgewählt aus der Gruppe bestehend aus SEQ ID Nr. 3, 6 und 9;
(f) einer Nukleotidsequenz, die ein Polypeptid kodiert mit einer Aminosäuresequenz, die zu mindestens 94% mit der Aminosäuresequenz identisch ist, die aus der Gruppe bestehend aus SEQ ID Nr. 3, 6 und 9 ausgewählt wurde;
(g) einer Nukleotidsequenz, die eine Variante einer DNA-Sequenz ist gemäß einer der Alternativen (a) bis (f), auf Grund der Entartung des genetischen Codes,
wobei stringente Bedingungen definiert sind als Hybridisierung in 4 x SSC bei 65°C und anschließendes wiederholtes Waschen in 0,1 x SSC bei 65°C für insgesamt etwa 1 Stunde.

2. Polypeptid, das von dem Nukleinsäuremolekül nach Anspruch 1 kodiert wird.

3. Vektor oder Expressionskassette, umfassend das Nukleinsäuremolekül nach Anspruch 1.

4. Zelle, die das Nukleinsäuremolekül nach Anspruch 1 als ein Transgen oder den Vektor oder die Expressionskassette nach Anspruch 3 umfasst.

5. Samen einer *Beta vulgaris*-Pflanze, **dadurch gekennzeichnet, dass** die Pflanze nicht *B. vulgaris* subsp. *maritima* ist, wobei der Samen das Nukleinsäuremolekül nach Anspruch 1 umfasst, wobei der Samen nicht ausschließlich durch ein im Wesentlichen biologisches Verfahren gewonnen wurde.

6. Samen einer *Beta vulgaris*-Pflanze nach Anspruch 5, wobei der Samen technisch behandelt wurde, wobei die technische Behandlung aus der Gruppe ausgewählt wird bestehend aus:
a) Polieren
b) Abrichten, vorzugsweise Pelletieren
c) Verkrustung
(d) Färbung.

7. Verfahren zur Erhöhung der Resistenz gegen einen Nematoden der Gattung Heterodera bei einer *Beta vulgaris*-Pflanze, einschließlich der folgenden Schritte:
(i) Integration des Nukleinsäuremoleküls nach Anspruch 1 mittels homologiegerichteter Reparatur in das Genom mindestens einer Zelle einer Pflanze, und Regeneration einer Pflanze aus der Pflanzenzelle; oder
(ii) Erhöhung der Expression des Nukleinsäuremoleküls nach Anspruch 1 in der Pflanze durch Modifikation des nativen Promotors oder durch Fusion des Nukleinsäuremoleküls mit einem heterologen Promotor, der im Vergleich zum nativen Promotor eine höhere Aktivität aufweist; oder
(iii) Umwandlung einer Pflanzenzelle mit dem Nukleinsäuremolekül nach Anspruch 1 oder dem Vektor oder der Expressionskassette nach Anspruch 3, und Regeneration der transgenen Pflanze aus der transformierten Pflanzenzelle.

8. Verfahren zur Erhöhung der Resistenz gegen einen Nematoden der Gattung Heterodera in einer *Beta vulgaris*-Pflanze nach Anspruch 7, wobei die homologie-gerichtete Reparatur durch eine ortsgerichtete Nuklease gefördert wird.

9. Verfahren zur Herstellung einer *Beta vulgaris*-Pflanze, die gegen einen Nematoden der Gattung Heterodera resistent ist, einschließlich der folgenden Schritte:
(a) Umwandlung einer Pflanzenzelle mit dem Nukleinsäuremolekül nach Anspruch 1, oder dem Vektor oder der Expressionskassette nach Anspruch 3; und
(b) Regeneration der transgenen Pflanze aus der transformierten Pflanzenzelle; oder
(i) Einführung einer ortsgerichteten Nuklease und einer Reparaturmatrix in eine Zelle einer Pflanze, wobei die ortsgerichtete Nuklease in der Lage ist, mindestens einen Einzelstrangbruch oder mindestens einen Doppelstrangbruch der DNA im Genom der Zelle stromaufwärts und/oder stromabwärts einer Zielregion zu erzeugen, und die Reparaturmatrix das Nukleinsäuremolekül gemäß Anspruch 1 umfasst;
(ii) Kultivierung der Zelle aus (i) unter Bedingungen, die eine homologie-gesteuerte Reparatur oder eine homologe Rekombination ermöglichen, wobei das Nukleinsäuremolekül aus der Reparaturmatrix in das Genom der Pflanze integriert wird; und
(iii) Regeneration einer Pflanze aus der unter (ii) modifizierten Zelle.

10. Verfahren nach Anspruch 9, **dadurch gekennzeichnet, dass** die Zielregion
(a) sich zwischen einem Marker gemäß SEQ ID Nr. 10 und einem Marker gemäß SEQ ID Nr. 11 befindet, oder
(b) von einem Marker nach SEQ ID Nr. 10 und einem Marker nach SEQ ID Nr. 11 flankiert wird, oder
(c) ein chromosomales Intervall zwischen einem Marker gemäß SEQ ID Nr. 10 und einem Marker gemäß SEQ ID Nr. 11 umfasst.

11. Verfahren nach Anspruch 9 oder 10, **dadurch gekennzeichnet, dass** der mindestens eine Einzelstrangbruch oder mindestens eine Doppelstrangbruch an einer Stelle auftritt, die höchstens 10.000 Basenpaare stromaufwärts und/oder stromabwärts der Zielregion liegt.

12. Verfahren zur Identifizierung und Selektion einer *Beta vulgaris*-Pflanze, die gegen einen Nematoden der Gattung Heterodera resistent ist, **dadurch gekennzeichnet, dass** das Verfahren mindestens einen Schritt (i) oder (ii) umfasst:
(i) Nachweis des Vorhandenseins und/oder der Expression des Nukleinsäuremoleküls gemäß Anspruch 1, oder des Vorhandenseins des Polypeptids gemäß Anspruch 2 in der *Beta vulgaris*-Pflanze oder einem Teil der *Beta vulgaris*-Pflanze; und/oder
(ii) Nachweis mindestens einer Region, die sich zwischen dem Marker gemäß SEQ ID Nr. 10 und dem Marker gemäß SEQ ID Nr. 11 befindet, von dem Marker gemäß SEQ ID Nr. 10 und dem Marker gemäß SEQ ID Nr. 11 flankiert wird, oder ein chromosomales Intervall zwischen dem Marker gemäß SEQ ID Nr. 10 und dem Marker gemäß SEQ ID Nr. 11 aufweist, Co-Segregation mit der Nukleotidsequenz des Nukleinsäuremoleküls nach Anspruch 1; und
(iii) Selektion der *Beta vulgaris*-Pflanze, die gegen einen Nematoden der Gattung Heterodera resistent ist,
wobei der Marker gemäß SEQ ID Nr. 10 ein einzelner Nukleotidpolymorphismus (SNP) an der Position 56940072 bp von Chromosom 5 ist, der auf den *Beta vulgaris*-Genotyp EL10 bezogen ist, wobei das Nukleotid ein T ist, und der Marker gemäß SEQ ID Nr. 11 ein einzelner Nukleotidpolymorphismus (SNP) an der Position 57809807 bp von Chromosom 5 ist, der auf den *Beta vulgaris*-Genotyp EL10 bezogen ist, wobei das Nukleotid ein T ist.

13. Verwendung eines Oligonukleotid-Primers mit einer Länge von mindestens 20 Nukleotiden, wobei das Oligonukleotid spezifisch als Sonde mit einer der ausgewählten Nukleotidsequenzen hybridisiert, bestehend aus der Gruppe
(i) einer Nukleotidsequenz, die die Sequenz umfasst, die aus der Gruppe ausgewählt wurde, bestehend aus SEQ ID Nr. 1, 4 und 7;
ii) einer Nukleotidsequenz, die die kodierende Sequenz umfasst, die aus der Gruppe bestehend aus SEQ ID Nr. 2, 5 und 8 ausgewählt wurde;
(iii) einer Nukleotidsequenz, die ein Polypeptid kodiert, mit einer Aminosäuresequenz, ausgewählt aus der Gruppe bestehend aus: SEQ ID Nr. 3, 6 und 9,
zur Amplifizierung einer Region, die für das Nukleinsäuremolekül nach Anspruch 1 spezifisch ist, in einer Polymerase-Kettenreaktion.

## Revendications

1. Molécule d'acide nucléique pour augmenter la résistance à un nématode du genre Heterodera dans une plante Beta vulgaris dans laquelle la molécule d'acide nucléique est exprimée, caractérisée de ce fait en ce que la molécule d'acide nucléique est sélectionnée dans le groupe constitué de :
(a) une séquence nucléotidique qui comprend la séquence sélectionnée dans le groupe constitué de : SEQ ID n° 1, 4 et 7 ;
(b) une séquence nucléotidique qui comprend la séquence de codage sélectionnée dans le groupe constitué de : SEQ ID n° 2, 5 et 8 ;
(c) une séquence nucléotidique qui s'hybride avec une séquence complémentaire d'une séquence nucléotidique selon (a), (b), (f) ou (g) sous des conditions stringentes ;
(d) une séquence nucléotidique qui comprend une séquence qui est au moins identique à 90 % à la séquence de la séquence nucléotidique de l'une quelconque de (a) ou (b) ;
(e) une séquence nucléotidique codant pour un polypeptide ayant une séquence d'acides aminés sélectionnée parmi le groupe constitué de : SEQ ID n° 3, 6 et 9 ;
(f) une séquence nucléotidique codant pour un polypeptide ayant une séquence d'acides aminés qui est au moins identique à 94 % à la séquence d'acides aminés sélectionnée parmi le groupe constitué de : SEQ ID n° 3, 6 et 9 ;
(g) une séquence nucléotidique qui est une variante d'une séquence d'ADN de l'une quelconque de (a) à (f) en raison de la dégénérescence du code génétique,
dans laquelle les conditions stringentes sont définies comme une hybridation dans 4 x SSC à 65 °C, et des lavages répétés ultérieurs dans 0,1 x SSC à 65 °C pendant environ 1 heure au total.

2. Polypeptide qui est codé par la molécule d'acide nucléique selon la revendication 1.

3. Vecteur ou cassette d'expression comprenant la molécule d'acide nucléique selon la revendication 1.

4. Cellule qui comprend la molécule d'acide nucléique selon la revendication 1 en tant que transgène, ou le vecteur ou la cassette d'expression selon la revendication 3.

5. Semence d'une plante Beta vulgaris, **caractérisée en ce que** la plante n'est pas *B. vulgaris* subsp. *maritima,* dans laquelle la semence comprend la molécule d'acide nucléique selon la revendication 1, dans laquelle la semence n'est pas exclusivement obtenue au moyen d'un processus essentiellement biologique.

6. Semence d'une plante Beta vulgaris selon la revendication 5, dans laquelle la semence a été techniquement traitée, de sorte que le traitement technique est sélectionné dans le groupe constitué de :
(a) Polissage
(b) Enrobage, de préférence pelliculage
(c) Incrustation
(d) Coloration.

7. Procédé pour augmenter la résistance à un nématode du genre Heterodera dans une plante Beta vulgaris, incluant les étapes suivantes :
(i) intégration de la molécule d'acide nucléique selon la revendication 1 au moyen d'une réparation dirigée par homologie dans le génome d'au moins une cellule d'une plante, et régénération d'une plante à partir de la cellule de la plante; ou
(ii) augmentation de l'expression de la molécule d'acide nucléique selon la revendication 1 dans la plante par modification du promoteur natif ou par fusion de la molécule d'acide nucléique avec un promoteur hétérologue qui présente une activité plus élevée en comparaison au promoteur natif ; ou
(iii) transformation d'une cellule végétale avec la molécule d'acide nucléique selon la revendication 1, ou le vecteur ou la cassette d'expression selon la revendication 3, et régénération de la plante transgénique à partir de la cellule végétale transformée.

8. Procédé pour augmenter la résistance à un nématode du genre Heterodera dans une plante Beta vulgaris selon la revendication 7, dans lequel la réparation dirigée par homologie est favorisée par une nucléase spécifique au site.

9. Procédé pour produire une plante Beta vulgaris ayant une résistance à un nématode du genre Heterodera, comprenant les étapes suivantes :
(a) transformation d'une cellule végétale avec la molécule d'acide nucléique selon la revendication 1, ou le vecteur ou la cassette d'expression selon la revendication 3 ; et
(b) régénération de la plante transgénique à partir de la cellule végétale transformée ; ou
(i) introduction d'une nucléase spécifique au site et d'une matrice de réparation dans une cellule d'une plante,
dans lequel la nucléase spécifique au site est susceptible de produire au moins une cassure simple brin ou au moins une cassure double brin de l'ADN dans le génome de la cellule en amont et/ou en aval d'une région cible et la matrice de réparation comprend la molécule d'acide nucléique selon la revendication 1 ;
(ii) culture de la cellule provenant de (i) sous des conditions qui permettent une réparation dirigée par homologie ou une recombinaison homologue, dans lequel la molécule d'acide nucléique est intégrée en provenance de la matrice de réparation jusque dans le génome de la plante ; et
(iii) régénération d'une plante à partir de la cellule modifiée en (ii).

10. Procédé selon la revendication 9, **caractérisé en ce que** la région cible
a) est située entre un marqueur selon SEQ ID n° 10 et un marqueur selon SEQ ID n° 11, ou
b) est flanquée par un marqueur selon SEQ ID n° 10 et un marqueur selon SEQ ID n° 11, ou
c) comprend un intervalle chromosomique entre un marqueur selon SEQ ID n° 10 et un marqueur selon SEQ ID n° 11.

11. Procédé selon la revendication 9 ou 10, **caractérisé en ce que** l'au moins une cassure simple brin ou l'au moins une cassure double brin se produit à une position qui est au plus 10 000 paires de bases en amont et/ou en aval de la région cible.

12. Procédé pour identifier et sélectionner une plante Beta vulgaris qui est résistante à un nématode du genre Heterodera, **caractérisé en ce que** le procédé inclut au moins l'étape (i) ou (ii) :
(i) détection de la présence et/ou de l'expression de la molécule d'acide nucléique selon la revendication 1, ou de la présence du polypeptide selon la revendication 2, dans la plante Beta vulgaris ou une portion de la plante Beta vulgaris ; et/ou
(ii) détection d'au moins une région qui est située entre le marqueur selon SEQ ID n° 10 et le marqueur selon SEQ ID n° 11, est flanquée par le marqueur selon SEQ ID n° 10 et le marqueur selon SEQ ID n° 11, ou comprend un intervalle chromosomique entre le marqueur selon SEQ ID n° 10 et le marqueur selon SEQ ID n° 11, en co-ségrégation avec la séquence nucléotidique de la molécule d'acide nucléique selon la revendication 1 ; et
(iii) sélection de la plante Beta vulgaris ayant une résistance à un nématode du genre Heterodera,
dans lequel le marqueur selon SEQ ID n° 10 est un polymorphisme mononucléotidique (SNP) à la position 56940072 bp du chromosome 5 référencé au génotype Beta vulgaris EL10, dans lequel ledit nucléotide est T, et le marqueur selon SEQ ID n° 11 est un polymorphisme mononucléotidique (SNP) à la position 57809807 bp du chromosome 5 référencé au génotype Beta vulgaris EL10, dans lequel ledit nucléotide est T.

13. Utilisation d'une amorce oligonucléotidique d'au moins 20 nucléotides de longueur, lequel oligonucléotide s'hybride de manière spécifique en tant que sonde avec l'une quelconque des séquences nucléotidiques sélectionnées dans le groupe constitué de
(i) une séquence nucléotidique qui comprend la séquence sélectionnée dans le groupe constitué de : SEQ ID n° 1, 4 et 7 ;
(ii) une séquence nucléotidique qui comprend la séquence de codage sélectionnée dans le groupe constitué de : SEQ ID n° 2, 5 et 8 ;
(iii) une séquence nucléotidique codant pour un polypeptide ayant une séquence d'acides aminés sélectionnée dans le groupe constitué de : SEQ ID n° 3, 6 et 9,
pour l'amplification d'une région spécifique à la molécule d'acide nucléique de la revendication 1 dans une réaction en chaîne par polymérase.
